# EUROPEAN PATENT SPECIFICATION

(11) **EP 1 611 098 B1**
(45) Date of publication and mention of the grant of the patent: **27.12.2006**
(21) Application number: 04725697.9
(22) Date of filing: 05.04.2004
(51) Int. Cl.: C07D 209/46, A61K 31/454, A61P 25/24

(54) **COMPOUNDS HAVING ACTIVITY AT 5HT2C RECEPTOR AND USES THEREOF**
VERBINDUNGEN MIT AKTIVITÄT AM 5HT2C-REZEPTOR UND ANWENDUNGEN DAVON
COMPOSES PRESENTANT UNE ACTIVITE PAR RAPPORT AU RECEPTEUR 5HT2C ET LEURS APPLICATIONS

(30) Priority: 07.04.2003 GB 0308025
(43) Date of publication of application: 04.01.2006
(73) Proprietor: GLAXO GROUP LIMITED, Greenford, Middlesex UB6 0NN (GB)
(72) Inventor: BONANOMI, Giorgio GlaxoSmithkline, I-37135 Verona (IT); HAMPRECHT, Dieter GlaxoSmithKline, I-37135 Verona (IT); MICHELI, Fabrizio GlaxoSmithKline, I-37135 Verona (IT); TERRENI, Silvia GlaxoSmithKline, I-37135 Verona (IT)
(74) Representative: Dolton, Peter Irving Ernest
(86) International application number: PCT/EP2004/003678
(87) International publication number: WO 2004/089897

(56) References cited:
- WO-A-03/057674
- WO-A-20/04041793
- US-A- 4 123 543
- US-A- 4 590 189
- PATENT ABSTRACTS OF JAPAN vol. 0051, no. 28 (C-067), 18 August 1981 (1981-08-18) -& JP 56 063961 A (KAMEYAMA TSUTOMU), 30 May 1981 (1981-05-30)

## Description

This invention relates to novel compounds having pharmacological activity, processes for their preparation, to compositions containing them and to their use in the treatment of CNS and other disorders.

WO 96/23783, WO 97/48699 and WO 97/48700 all disclose a series of indoline derivatives which are 5-HT_{2C} receptor antagonists and which are claimed to be useful in the treatment of various CNS disorders.

A novel class of compounds possessing 5-HT_{2C} receptor activity has been found. The present invention therefore provides, in a first aspect, a compound of formula (I) or a pharmaceutically acceptable salt thereof: 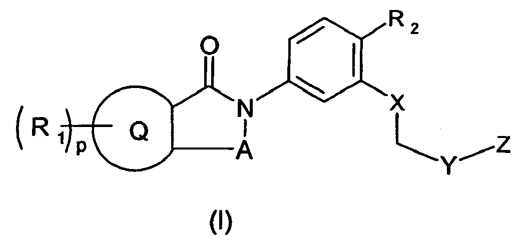 wherein:
R₁ is halogen, cyano, C₁₋₆alkyl, C₃₋₇cycloalkyl, C₃₋₇cycloalkyloxy, C₁₋₆alkoxy, C₁₋₆alkylthio, hydroxy, amino, mono- or di-C₁₋₆alkylamino, an N-linked 4 to 7 membered heterocyclic group, nitro, haloC₁₋₆alkyl, haloC₁₋₆alkoxy, aryl, -COOR₃, -COR₄ (wherein R₃ and R₄, are independently hydrogen or C₁₋₆alkyl) or -COR₅ (wherein R₅ is amino, mono- or di-C₁₋₆alkylamino or an N-linked 4 to 7 membered heterocyclic group);
p is 0, 1 or 2 or 3;
Q is a 6- membered aromatic group or a 6-membered heteroaromatic group;
A is -(CH₂-CH₂)-, -(CH=CH)-, or a group -(CHR₇)- wherein R₇ is hydrogen, halogen, hydroxy, cyano, nitro, C₁₋₆alkyl, C₃₋₇cycloalkyl, C₃₋₇cycloalkyloxy, haloC₁₋₆alkyl, C₁₋₆ alkoxy, haloC₁₋₆alkoxy or C₁₋₆alkylthio;
R₂ is hydrogen, halogen, hydroxy, cyano, nitro, C₁₋₆alkyl, C₁₋₆alkanoyl, C₃₋₇cycloalkyl, C₃₋₇cycloalkyloxy, haloC₁₋₆alkyl, C₁₋₆alkoxy, haloC₁₋₆alkoxy, C₁₋₆alkylthio, amino, mono- or di-C₁₋₆alkylamino or an N-linked 4 to 7 membered heterocyclic group;
X is oxygen, sulfur, -CH₂- or NR₈ wherein R₈ is hydrogen or C₁₋₆alkyl;
Y is a single bond, -CH₂-, -(CH₂)₂- or -CH=CH-; and
Z is an optionally substituted N-linked heterocyclic group or a C-linked 4 to 7 membered heterocyclic group containing at least one nitrogen, or Z is -NR₉R₁₀ where R₉ and R₁₀ are independently hydrogen or C₁₋₆alkyl.

The following terms, whether used alone or as part of another group are to be given the following meanings, unless otherwise stated.

The term "halogen" and its abbreviated form "halo" are used herein to describe fluorine, chlorine, bromine or iodine.

The term "alkyl" is used herein to describe a straight chain or branched fully saturated hydrocarbon group. "C₁₋₆alkyl" refers to alkyl groups having from one to six carbon atoms, including all isomeric forms, such as methyl, ethyl, propyl, isopropyl, butyl, isobutyl, sec-butyl, tert-butyl, pentyl, neopentyl, sec-pentyl, n-pentyl, isopentyl, tert-pentyl and hexyl.

The term "C₁₋₆alkanoyl" refers to an alkanoyl group having from 1 to 6 carbon atoms, such as methanoyl (or "formyl"), ethanoyl (or "acetyl"), propanoyl, isopropanoyl, butanoyl, isobutanoyl, sec-butanoyl, pentanoyl, neopentanoyl, sec-pentanoyl, isopentanoyl, tertpentanoyl and hexanoyl.

The term "C₁₋₆alkoxy" refers to a straight chain or branched chain alkoxy (or "alkyloxy") group having from one to six carbon atoms, including all isomeric forms, such as methoxy, ethoxy, propoxy, isopropoxy, butoxy, isobutoxy, sec-butoxy, tert-butoxy, pentoxy, neopentoxy, sec-pentoxy, n-pentoxy, isopentoxy, tert-pentoxy and hexoxy.

The term "C₃₋₇cycloalkyl" refers to a cycloalkyl group consisting of from 3 to 7 carbon atoms, such as cyclopropane, cyclobutane, cyclopentane, cyclohexane and cycloheptane. Optional substituents for C₃₋₇cycloalkyl includes one or more halogen, hydroxy, oxo, C₁₋₆alkyl, cyano, CF₃, OCF₃, C₁₋₆alkoxy and C₁₋₆alkanoyl.

The term "C₁₋₆alkylthio" refers to a straight chain or branched chain alkylthio group having from one to six carbon atoms, such as methylthio, ethylthio, propylthio, isopropylthio, butylthio, isobutylthio, sec-butylthio, tert-butylthio, pentylthio, neopentylthio, sec-pentylthio, n-pentylthio, isopentylthio, tert-pentylthio and hexylthio.

The term "mono- or di-C₁₋₆alkylamino" refers to an amino group which is substituted by one C₁₋₆alkyl group or an amino group which is substituted by two C₁₋₆alkyl groups, the two amino groups being the same or different. Examples of monoC1-6alkylamino include methylamine, ethylamine, propylamine, isopropylamine, butylamine, isobutylamine, sec-butylamine, tert-butylamine, pentylamine, neopentylamine, sec-pentylamine, n-pentylamine, isopentylamine, tert-pentylamine and hexylamine. Examples of di-C1-6alkylamino include dimethylamine, diethylamine, dipropylamine, diisopropylamine, dibutylamine, diisobutylamine, disec-butylamine, ditert-butylamine, dipentylamine, dineopentylamine, dihexylamine, butylmethylamino, isopropylmethylamino, ethylisopropylamino, ethylmethylamino, etc.

The term "5- or 6- membered aromatic or heteroaromatic group" is used herein to describe groups such as phenyl, pyrrolyl, pyrrolinyl, pyrazolinyl, imidazolyl, pyrazolyl, oxadiazolyl, isothiazolyl, thiazolyl, pyridyl, thiazinyl, pyridazinyl, pyrimidinyl and pyrazinyl.

The term "aryl" is used herein to describe 5- or 6- membered aromatic or heteroaromatic groups such as phenyl, pyrrolyl, pyrrolinyl, pyrazolinyl, imidazolyl, pyrazolyl, oxadiazolyl, isothiazolyl, thiazolyl, pyridyl, thiazinyl, pyridazinyl, pyrimidinyl and pyrazinyl, as well as naphthyl and other bicyclic heteroaromatic groups. These groups may be optionally substituted by one or more of C₁₋₆alkyl (to form "arylC₁₋₆alkyl"), halogen, CF₃ or C₁₋₆ alkoxy (to form "arylC₁₋₆alkoxy").

The terms "halo C₁₋₆alkoxy" or "haloC₁₋₆alkyl" are used to describe a C₁₋₆alkoxy or a C₁₋₆alkyl group, respectively, substituted with one or more halogens. Examples include -CHCl₂, -CF₃, -OCF₃, etc.

The term "heterocyclic group" is used herein to describe a stable aromatic or non-aromatic ring containing 1, 2 or 3 heteroatoms selected from nitrogen, sulphur and oxygen. Suitable examples of 4 to 7 membered heterocyclic groups include azetidinyl, pyrrolidinyl, imidazolidinyl, pyrazolidinyl, oxazolinyl, isothiazolidinyl, thiazolidinyl, pyrrolyl, pyrrolinyl, pyrazolinyl, imidazolyl, pyrazolyl, isothiazolyl, thiazolyl, piperidyl, piperazinyl, morpholinyl, pyridyl, pyridazinyl, pyrimidinyl, pyrazinyl, azepinyl, azepanyl, dioxolanyl, thienyl, tetrahydrothienyl, tetrahydrofuryl, dioxanyl and dithianyl.

The term "N-linked heterocyclic group" is used herein to describe a heterocyclic groupwhich is linked to the remainder of the molecule via a nitrogen atom. Suitable examples of 4 to 7 membered N-linked heterocyclic groups include azetidinyl, pyrrolidinyl, imidazolidinyl, pyrazolidinyl, oxazolinyl, isothiazolidinyl, thiazolidinyl, pyrrolyl, pyrrolinyl, pyrazolinyl, imidazolyl, pyrazolyl, piperidyl, piperazinyl, morpholinyl and azepanyl.

The term "C-linked heterocyclic group containing at least one nitrogen" is used herein to describe a heterocyclic group which is contains at least one nitrogen atom and is linked to the remainder of the molecule via a carbon atom. Suitable examples of 4 to 7 membered C-linked heterocyclic groups containing at least one nitrogen include azetidinyl, pyrrolidinyl, imidazolidinyl, pyrazolidinyl, pyrrolyl, pyrrolinyl, pyrazolinyl, imidazolyl, pyrazolyl, piperidyl, piperazinyl, morpholinyl, pyridyl, pyridazinyl, pyrimidinyl, pyrazinyl, azepinyl and azepanyl.

The term "optionally substituted N-linked or C-linked 4 to 7 membered heterocyclic group" means that more than one optional substituent may be present in the N-linked or C-linked 4 to 7 membered heterocyclic group, which may be the same or different, and may be attached to any carbon atom of the heterocycle or an available nitrogen atom.

Suitable optional substituents for the N-linked or C-linked heterocycle include C₁₋₆alkyl, amino, mono- or di- C₁₋₆alkylamino, aryl, arylC₁₋₆alkyl, arylamino, hydroxy, C₁₋₆alkylamido, hydroxyC₁₋₆alkyl, C₁₋₆alkoxycarbonyl, halogen, haloC1-6alkyl, a heteroaromatic group (such as indole or benzimidazole), an aromatic or non-aromatic N-linked or C-linked heterocycle or an aromatic or non-aromatic heterocycleC₁₋₆alkyl optionally substituted by C₁₋₆alkyl. Examples of aromatic or non-aromatic heterocycleC₁₋₆ alkyl include heterocycle-methyl (such as pyridinyl-methyl and benzimidazolyl-methyl) and heterocycle-ethyl (such as morpholinyl-ethyl and indolyl-ethyl).

Substituents in the N-linked or C-linked heterocycle may form a bridge structure, to form a group such as for example 2-oxa-5-azabicyclo[2.2.1]heptyl. Such a bicyclic group may be further substituted by the substituents listed above. More than one substituent may be present on the same carbon atom to form spiro structures such as 1,4 and 1,5 dioxa spiro compounds.

When p is 2 or 3, R₁ may be the same or or different.

When A is a group -(CHR₇)-, preferably R₇ is hydrogen. Preferably A is CH₂-.

Preferably Q is phenyl, as follows:

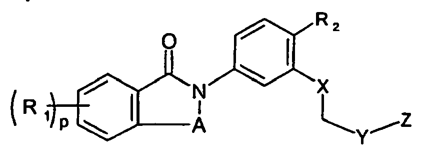

Preferably p is 1, 2 or 3 and R₁ is/are halogen (particularly chloro or fluoro), C₁₋₆alkyl (particularly methyl) or CF₃.

If R₁ is attached at the position marked below with an asterisk, then R₁ is preferably fluoro:

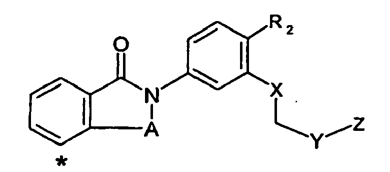

When Q is phenyl and p is 1, preferably R₁ is attached at the position marked below with an asterisk:

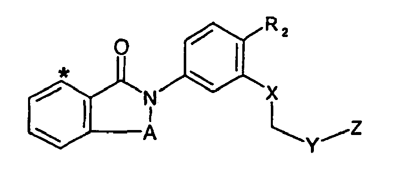

When Q is phenyl and p is 2 or 3, preferably R₁ is attached at two or more of the positions marked below with arrows:

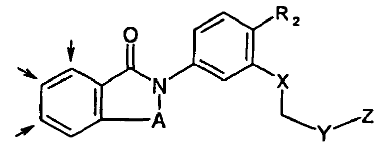

Preferably R₂ is C₁₋₆alkoxy (particularly methoxy).

Preferably X is oxygen.

Preferably Y is -CH₂-.

Preferably Z is an optionally substituted N-linked 4 to 7 membered heterocycle, in particular optionally substituted piperidyl. Preferred substituents include halogen (particularly fluoro) and C₁₋₆alkyl (particularly methyl).

Preferred compounds are compounds of formula (Ia): 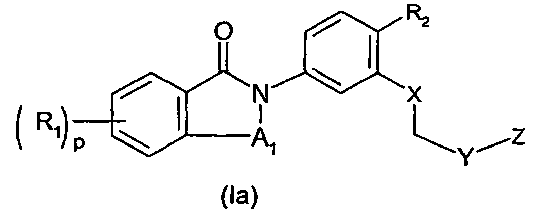 wherein R₁, p, R₂, X, Y, Z, are as defined for formula (I) and A₁ is -CH₂- or -HC(Me)-. Preferred features of formula (I) also apply to formula (Ia).

Preferred compounds include:
- 2-[4-Methoxy-3-(2-piperidin-1-yl-ethoxy)phenyl]-2,3-dihydroisoindol-1-one
- 6-Fluoro-2-[4-methoxy-3-(2-piperidin-1-yl-ethoxy)phenyl]-2,3-dihydroisoindol-1-one
- 7-Bromo-2-{4-methoxy-3-[2-(4-methyl-piperidin-1-yl)-ethoxy]phenyl}-2,3-dihydroisoindol-1-one hydrochloride
- 7-Chloro-2-{4-methoxy-3-[2-(4-methyl-piperidin-1-yl)-ethoxy]phenyl}-3-methyl-2,3-dihydroisoindol-1-one
- 2-{4-Methoxy-3-[2-(4-methyl-piperidin-1-yl)-ethoxy]phenyl}-7-trifluoromethyl-2,3-dihydroisoindol-1-one
- 5,7-Dichloro-2-{4-methoxy-3-[2-(4-methyl-piperidin-1-yl)-ethoxy]phenyl}-2,3-dihydroisoindol-1-one
- 7-Chloro-2-[4-methoxy-3-(2-piperidin-1-yl-ethoxy)phenyl]-2,3-dihydroisoindol-1-one
- 6-Chloro-2-[4-methoxy-3-(2-piperidin-1-yl-ethoxy)phenyl]-2,3-dihydroisoindol-1-one hydrochloride
- 5-Chloro-2-[4-methoxy-3-(2-piperidin-1-yl-ethoxy)phenyl]-2,3-dihydroisoindol-1-one
- 5,7-Dichloro-2-{4-methoxy-3-[2-(*cis*-2,6-dimethyl-piperidin-1-yl)-ethoxy]phenyl}-2,3-dihydroisoindol-1-one
- 7-Chloro-2-{4-methoxy-3-[2-(4-methyl-piperidin-1-yl)-ethoxy]phenyl}-2,3-dihydroisoindol-1-one
- 6-Chloro-2-{4-methoxy-3-[2-(4-methyl-piperidin-1-yl)-ethoxy]phenyl}-2,3-dihydroisoindol-1-one
- 5-Chloro-2-{4-methoxy-3-[2-(4-methyl-piperidin-1-yl)-ethoxy]phenyl}-2,3-dihydroisoindol-1-one
- 7-Methyl-2-{4-methoxy-3-[2-(4-methyl-piperidin-1-yl)-ethoxy]phenyl}-2,3-dihydroisoindol-1-one
- 6,7-Difluoro-2-{4-methoxy-3-[2-(4-methyl-piperidin-1-yl)-ethoxy]phenyl}-2,3-dihydroisoindol-1-one
- 5,6-Dichloro-2-(4-methoxy-3-[2-(4-methyl-piperidin-1-yl)-ethoxy]phenyl)-2,3-dihydroisoindol-1-one
- 7-Fluoro-2-{4-methoxy-3-[2-(piperidin-1-yl)-ethoxy]phenyl}-2,3-dihydroisoindol-1-one
- 4-Fluoro-2-{4-methoxy-3-[2-(piperidin-1-yl)-ethoxy]phenyl}-2,3-dihydroisoindol-1-one
- 5,7-Dimethyl-2-{4-methoxy-3-[2-(4-methyl-piperidin-1-yl)-ethoxy]phenyl}-2,3-dihydroisoindol-1-one
- 6,7-Dichloro-2-{4-methoxy-3-[2-(4-methyl-piperidin-1-yl)-ethoxy]phenyl}-2,3-dihydroisoindol-1-one
- 5-Fluoro-2-{4-methoxy-3-[2-(4-methyl-piperidin-1-yl)-ethoxy]phenyl}-7-trifluoromethyl-2,3-dihydroisoindol-1-one
- 7-Chloro-4,5-difluoro-2-{4-methoxy-3-[2-(4-methyl-piperidin-1-yl)-ethoxy]phenyl}-2,3-dihydroisoindol-1-one
- 4-Fluoro-2-{4-methoxy-3-[2-(4-methyl-piperidin-1-yl)-ethoxy]phenyl}- 7-trifluoromethyl-2,3-dihydroisoindol-1-one
- 4-Fluoro-2-{4-methoxy-3-[2-(4-methyl-piperidin-1-yl)-ethoxy]phenyl}- 7-trifluoromethyl-2,3-dihydroisoindol-1-one
- 4-Fluoro-2-{4-methoxy-3-[2-(4-methyl-piperidin-1-yl)-ethoxy]phenyl}- 7-trifluoromethyl-2,3-dihydroisoindol-1-one
- 4-Fluoro-2-{4-methoxy-3-[2-(4-methyl-piperidin-1-yl)-ethoxy]phenyl}- 7-trifluoromethyl-2,3-dihydroisoindol-1-one
- 5,7-Dichloro-2-{4-methoxy-3-[2-(4,4-dimethyl-piperidin-1-yl)-ethoxy]phenyl}-2,3-dihydroisoindol-1-one 5,7-Dichloro-2-{4-methoxy-3-[2-(azepan-1-yl)-ethoxy]-phenyl}-2,3-dihydroisoindol-1-one
- 5,7-Dichloro-2-{4-methoxy-3-[2-(2-methyl-piperidin-1-yl)-ethoxy]phenyl}-2,3-dihydroisoindol-1-one
- 6-{4-Methoxy-3-[2-(4-methyl-piperidin-1-yl)-ethoxy]-phenyl}-2-methyl-4-trifluoromethyl-6,7-dihydro-pyrrolo[3,4-*b*]pyridin-5-one
- 5,7-Dichloro-4-fluoro-2-{4-methoxy-3-[2-(4-methyl-piperidin-1-yl)-ethoxy]phenyl}-2,3-dihydroisoindol-1-one
and pharmaceutically acceptable salts thereof.

The compounds of formula (I) can form acid addition salts. It will be appreciated that for use in medicine the salts of the compounds of formula (I) should be pharmaceutically acceptable. Suitable pharmaceutically acceptable salts will be apparent to those skilled in the art and include those described in J. Pharm. Sci., 1977, 66, 1-19, such as acid addition salts formed with inorganic acids e.g. hydrochloric, hydrobromic, sulfuric, nitric or phosphoric acid; and organic acids e.g. succinic, maleic, acetic, fumaric, citric, tartaric, benzoic, p-toluenesulfonic, methanesulfonic or naphthalenesulfonic acid.

The compounds of this invention may be in crystalline or non-crystalline form, and, if crystalline, may optionally be hydrated or solvated. This invention includes within its scope stoichiometric hydrates as well as compounds containing variable amounts of water.

Certain compounds of formula (I) are capable of existing in stereoisomeric forms (e.g. geometric or *("cis-trans")* isomers, diastereomers and enantiomers) and the invention extends to each of these stereoisomeric forms and to mixtures thereof including racemates. The different stereoisomeric forms may be separated one from the other by the usual methods, or any given isomer may be obtained by stereospecific or asymmetric synthesis. The invention also extends to any tautomeric forms and mixtures thereof.

The present invention also provides a process for the preparation of a compound of formula (I) or a pharmaceutically acceptable salt thereof, which process comprises:
(a) reacting a compound of formula (II): 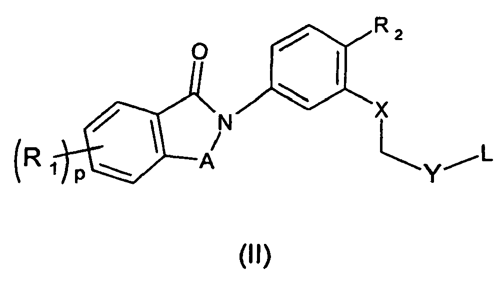 wherein R₁, R₂, p, A, X, and Y are as defined for formula (I), and L is a leaving group, with a compound of formula (III):

   Z-H (III)

   wherein Z is as defined for formula (I); or
(b) reacting a compound of formula (IV): 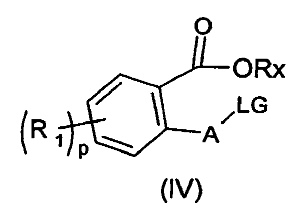 wherein Rx is alkyl and LG is a suitable leaving group, with a compound of formula (V) or a corresponding salt: 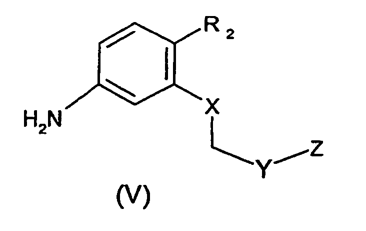 or
(c) reacting a compound of formula (VI): 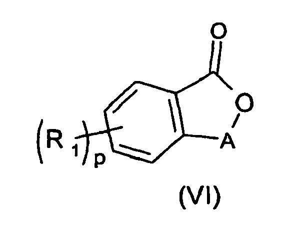
with a compound of formula (V) in the presence of AlMe₃ or a similar oxophilic reagent followed by treatment of the resulting amide under dehydrating conditions, e.g. with PPh₃ and dialkylazadicarboxylate;
and thereafter, for either process (a), process (b) or process (c), optionally followed by:
- removing any protecting groups; and/or
- converting a compound of formula (I) into another compound of formula (I); and/or
- forming a pharmaceutically acceptable salt.

For the reaction of process (a), suitably L is mesylate. The reaction may take place in a solvent such as DMF in the presence of sodium iodide and potassium carbonate.

For the reaction of process (b), LG is a leaving group such as halogen, for example chloro or bromo, or a sulfonate ester, for example mesyl or tosyl. Rx is alkyl, for example Me. The reaction may take place in a solvent such as DMF at elevated temperature, suitably 150 °C in a microwave reactor. Where this reaction does not lead to spontaneous cyclisation of the intermediate alkylation product this material is treated with AlMe₃ or a similar oxophilic reagent.

Compounds of formula (I) can be converted into further compounds of formula (I) using standard techniques. For example, and by way of illustration rather than limitation, a compound wherein A is -(HCOH)- may be converted to a compound wherein A is -(CH₂)-by using a suitable reducing agent such as triethylsilane-trifluoroacetic acid using dichloromethane as solvent.

Compounds of formulae (II), (III) and (IV) are commercially available or may be prepared according to methods described herein or may be prepared according to known methods or by analogous methods thereto.

Those skilled in the art will appreciate that it may be necessary to protect certain groups to carry out the above processes. Suitable protecting groups and methods for their attachment and removal are conventional in the art of organic chemistry, such as those described in Greene T.W. 'Protective groups in organic synthesis' New York, Wiley (1981).

Pharmaceutically acceptable salts may be prepared conventionally by reaction with the appropriate acid or acid derivative.

In another aspect, the present invention provides a pharmaceutical composition comprising a compound of formula (I) or a pharmaceutically acceptable salt thereof, and a pharmaceutically acceptable carrier or excipient.

In a further aspect, the present invention provides a process for preparing a pharmaceutical composition, the process comprising mixing a compound of formula (I) or a pharmaceutically acceptable salt thereof and a pharmaceutically acceptable carrier or excipient.

A pharmaceutical composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusible solutions or suspensions or suppositories. Orally administrable compositions are generally preferred.

Tablets and capsules for oral administration may be in unit dose form, and may contain conventional excipients, such as binding agents (e.g. pregelatinised maize starch, polyvinylpyrrolidone or hydroxypropyl methylcellulose);, fillers (e.g. lactose, microcrystalline cellulose or calcium hydrogen phosphate);, tabletting lubricants lubricants (e.g. magnesium stearate, talc or silica);, disintegrants (e.g. potato starch or sodium starch glycollate); and acceptable wetting agents (e.g. sodium lauryl sulphate). The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents (e.g. sorbitol syrup, cellulose derivatives or hydrogenated edible fats), emulsifying agents (e.g. lecithin or acacia), non-aqueous vehicles (which may include edible oils e.g. almond oil, oily esters, ethyl alcohol or fractionated vegetable oils), preservatives (e.g. methyl or propyl-p-hydroxybenzoates or sorbic acid), and, if desired, conventional flavourings or colorants, buffer salts and sweetening agents as appropriate. Preparations for oral administration may be suitably formulated to give controlled release of the active compound.

For parenteral administration, fluid unit dosage forms are prepared utilising a compound of the invention or pharmaceutically acceptable salt thereof and a sterile vehicle. Formulations for injection may be presented in unit dosage form e.g. in ampoules or in multi-dose, utilising a compound of the invention or pharmaceutically acceptable salt thereof and a sterile vehicle, optionally with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilisation cannot be accomplished by filtration. The compound can be sterilised by exposure to ethylene oxide before suspension in a sterile vehicle. Advantageously, a surfactant or wetting agent is included in the composition to facilitate uniform distribution of the compound.

Lotions may be formulated with an aqueous or oily base and will in general also contain one or more emulsifying agents, stabilising agents, dispersing agents, suspending agents, thickening agents, or colouring agents. Drops may be formulated with an aqueous or non-aqueous base also comprising one or more dispersing agents, stabilising agents, solubilising agents or suspending agents. They may also contain a preservative.

The compounds of the invention may also be formulated in rectal compositions such as suppositories or retention enemas, e.g. containing conventional suppository bases such as cocoa butter or other glycerides.

The compounds of the invention may also be formulated as depot preparations. Such long acting formulations may be administered by implantation (for example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

For intranasal administration, the compounds of the invention may be formulated as solutions for administration via a suitable metered or unitary dose device or alternatively as a powder mix with a suitable carrier for administration using a suitable delivery device. Thus compounds of formula (I) may be formulated for oral, buccal, parenteral, topical (including ophthalmic and nasal), depot or rectal administration or in a form suitable for administration by inhalation or insufflation (either through the mouth or nose).

The compounds of the invention may be formulated for topical administration in the form of ointments, creams, gels, lotions, pessaries, aerosols or drops (e.g. eye, ear or nose drops). Ointments and creams may, for example, be formulated with an aqueous or oily base with the addition of suitable thickening and/or gelling agents. Ointments for administration to the eye may be manufactured in a sterile manner using sterilised components.

The compounds of the present invention have affinity for the 5-HT_{2C} receptor. The affinity can be determined by assessing their ability to displace [³H]-mesulergine from rat or human 5-HT_{2C} clones expressed in 293 cells *in vitro,* as described in WO 94/04533.

All the Example compounds were tested according to this assay and were found to have pKi values >5.8. Some compounds show a considerably higher affinity in the range of 7.0 to >9.0 in human cells.

The intrinsic activity of the compounds of this invention can be determined according to the [³⁵S]GTPγS functional assay which is described in WO 99/07700.

Compounds of formula (I) and their pharmaceutically acceptable salts are of use in the treatment of certain CNS disorders such as depression (which term is used herein to include bipolar depression, unipolar depression, single or recurrent major depressive episodes with or without psychotic features, catatonic features, melancholic features, atypical features or postpartum onset, seasonal affective disorder, dysthymic disorders with early or late onset and with or without atypical features, neurotic depression and social phobia, depression accompanying dementia for example of the Alzheimer's type, vascular dementia with depressed mood, schizoaffective disorder or the depressed type, and depressive disorders resulting from general medical conditions including, but not limited to, myocardial infarction, diabetes, miscarriage or abortion, *etc*), anxiety including generalised anxiety and social anxiety disorder, schizophrenia, panic disorder, agoraphobia, social phobia, epilepsy, obsessive compulsive disorder and post-traumatic stress disorder, pain (particularly neuropathic pain), migraine, memory disorders, including dementia, amnesic disorders and age-associated memory impairment, disorders of eating behaviours including anorexia nervosa and bulimia nervosa, sexual dysfunction, sleep disorders (including disturbances of circadian rhythm, dyssomnia, insomnia, sleep apnea and narcolepsy), withdrawal from abuse of drugs such as of cocaine, ethanol, nicotine, benzodiazepines, alcohol, caffeine, phencyclidine (phencyclidine-like compounds), opiates (e.g. cannabis, heroin, morphine), sedative ipnotic, amphetamine or amphetamine-related drugs (e.g. dextroamphetamine, methylamphetamine) or a combination thereof, Alzheimer's disease, motor disorders such as Parkinson's disease, dementia in Parkinson's disease, neuroleptic-induced Parkinsonism and tardive dyskinesias, as well as other psychiatric disorders, disorders associated with spinal trauma and/or head injury such as hydrocephalus, gastrointestinal disorders such as IBS (Irritable Bowel Syndrome), Crohn's disease, ulcerative colitis, non-steroidal antiinflammatory drug induced damage) as well as microvascular diseases such as macular oedema and retinopathy.

It is to be understood that, as used herein, the term "treatment" refers to alleviation of established symptoms as well as prophylaxis.

Thus the present invention also provides a compound of formula (I) or a pharmaceutically acceptable salt thereof, for use as a therapeutic substance. In particular, the present invention provides a compound of formula (I) or a pharmaceutically acceptable salt thereof for use in the treatment of the above disorders. In particular the invention provides a compound of formula (I) or a pharmaceutically acceptable salt thereof for use as a therapeutic substance in the treatment of a CNS disorder. Preferably the CNS disorder is depression or anxiety.

Compounds of the invention may be administered in combination with other active substances such as 5HT3 antagonists, NK-1 antagonists, serotonin agonists, selective serotonin reuptake inhibitors (SSRI), noradrenaline re-uptake inhibitors (SNRI), tricyclic antidepressants or dopaminergic antidepressants.

Suitable 5HT3 antagonists which may be used in combination of the compounds of the inventions include for example ondansetron, granisetron, metoclopramide.

Suitable serotonin agonists which may be used in combination with the compounds of the invention include sumatriptan, rauwolscine, yohimbine, metoclopramide.

Suitable SSRIs which may be used in combination with the compounds of the invention include fluoxetine, citalopram, femoxetine, fluvoxamine, paroxetine, indalpine, sertraline, zimeldine.

Suitable SNRIs which may be used in combination with the compounds of the invention include venlafaxine and reboxetine.

Suitable tricyclic antidepressants which may be used in combination with a compound of the invention include imipramine, amitriptiline, chlomipramine and nortriptiline.

Suitable dopaminergic antidepressants which may be used in combination with a compound of the invention include bupropion and amineptine.

It will be appreciated that the compounds of the combination or composition may be administered simultaneously (either in the same or different pharmaceutical formulations), separately or sequentially.

In another aspect, the invention provides for the use of a compound of formula (I) or a pharmaceutically acceptable salt thereof in the manufacture of a medicament for use in the treatment of the above disorders. In particular the present invention provides the use of a compound of formula (I) or a pharmaceutically acceptable salt thereof in the manufacture of a medicament for use in the treatment of a CNS disorder. Preferably the CNS disorder is depression or anxiety.

The composition of the present invention may contain from 0.1% to 99% by weight, preferably from 10 to 60% by weight, of the active material, depending on the method of administration. The dose of the compound used in the treatment of the aforementioned disorders will vary in the usual way with the seriousness of the disorders, the weight of the sufferer, and other similar factors. However, as a general guide suitable unit doses may be 0.05 to 1000 mg, more suitably 1.0 to 200 mg, and such unit doses may be administered more than once a day, for example two or three times a day. Such therapy may extend for a number of weeks or months. When administered in accordance with the invention, no unacceptable toxicological effects are expected with the compounds of the invention.

All publications, including but not limited to patents and patent applications, cited in this specification are herein incorporated by reference as if each individual publication were specifically and individually indicated to be incorporated by reference herein as though fully set forth.

The following Descriptions and Examples illustrate the preparation of compounds of the present invention.

### General Procedure 1 (G1): Hydrochloride salt formation

To a solution of the free base in CH₂Cl₂ was added excess HCl (1M in Et₂O). The resulting mixture was evaporated to dryness and the residue triturated using a mixture of EtOAc:Et₂O ca. 1:1 and dried to give the respective hydrochloride salt.

### General Procedure 2 (G2): Bromination to form substituted benzyl bromides

A mixture of the substituted toluene, *N*-bromosuccinimide (typically 1.1 eq.) and benzoyl peroxide (0.05 eq.) in dry CCl₄ (2 ml per mmol substituted toluene) was heated at reflux.

After complete conversion (typically 5 h; NMR control) the mixture was allowed to cool, filtered (washing with Et₂O), concentrated and submitted to column chromatography to give the respective substituted benzyl bromide.

### General Procedure 3 (G3): Methyl ester formation

To the substituted benzoic acid in dry CH₂Cl₂ (ca. 0.2-0.5 M) containing catalytic DMF (ca. 0.1 eq.) at 0 °C was added with stirring oxalyl chloride (1.3 eq.). The ice bath was removed. After gas evolution had ceased (typically 1-2 h) volatiles were removed *in vacuo.* To the residue was added dry methanol (to give a ca. 1 M solution; CAUTION: on scales above ca. 5 mmol this involved a significant exotherme) and the mixture was kept for 16 h. The material obtained after evaporation of the volatiles was used as such or purified by column chromatography.

### General Procedure 4 (G4): Chlorination to form substituted benzyl chlorides

A mixture of the substituted toluene, *N*-chlorosuccinimide (typically 1.1 eq.) and benzoyl peroxide (0.05 eq.) in dry CCl₄ (2 ml per mmol substituted toluene) was heated at reflux. After complete conversion (typically 5 h; NMR control) the mixture was allowed to cool, filtered (washing with Et₂O), concentrated and submitted to column chromatography to give the respective substituted benzyl chloride.

### Preparation 1 (P1): 1-[2-(2-Methoxy-5-nitro-phenoxy)-ethyl]-piperidine

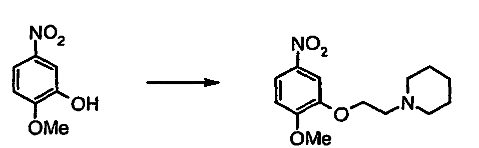

To a solution of 2-methoxy-5-nitrophenol (10 g) in DMF (45 ml) at room temperature were added K₂CO₃ (23 g) and 1-(2-chloroethyl)piperidin hydrochloride (12 g). The suspension was stirred at room temperature for 3 days. The suspension was diluted with water and twice extracted with EtOAc: Et₂O 1:2, the combined organic phases were washed with brine, concentrated to dryness *in vacuo,* to obtain the title product as a brown oil (99%).
**NMR (¹H, CDCl₃):** δ 7.85 (dd, 1H), 7.75 (d, 1H), 6.90 (d, 1H), 4.20 (t, 2H), 3.90 (s, 3H), 2.80 (t, 2H), 2.45-2.60 (m, 4H), 1.55-1.65 (m, 4H) (m, 4H), 1.35-1.50 (m, 2H).
MS *(mlz):* 281[MH]⁺.

### Preparation 2 (P2): 4-Methoxy-3-(2-piperidin-1-yl-ethoxy)-phenylamine (hydrochloride)

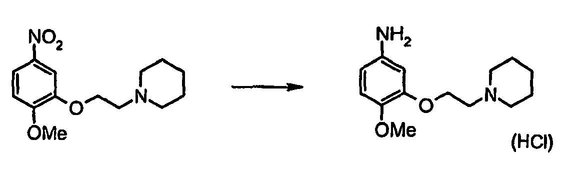

To 1-[2-(2-methoxy-5-nitro-phenoxy)-ethyl]-piperidine (59 mmol) in a mixture of MeOH (140 ml), NH₄Cl (17.1 g), water (140 ml) and conc. aqueous HCl (4.9 ml) was added iron powder (10.6 g). The mixture was heated at reflux with vigorous stirring for 90 min. and concentrated to *ca.* half volume *in vacuo.* EtOAc was added, filtered (Celite), washing with water. Et₂O was added, the layers were mixed and the aqueous layer collected. K₂CO₃ was added until pH ca. 9-10 and the mixture was twice extracted with EtOAc:Et₂O 1:1. The combined organic layers were dried (K₂CO₃), filtered and concentrated to give the free base of the title compound as a red oil (56 mmol). **NMR (¹H, CDCl₃)**: δ 6.67 (d, 1H), 6.30 (d, 1H), 6.20 (dd, 1H), 4.07 (t, 2H), 3.75 (s, 3H), 3.40 (bs, 2H), 2.80 (t, 2H), 2.40-2.55 (m, 4H), 1.51-1.62 (m, 4H), 1.35-1.46 (m, 2H).
Batches of this material were converted to the hydrochloride salt as required by treating a solution of the free base in dichloromethane with 1 eq. HCl (1 M solution in Et₂O), followed by evaporation to dryness.

### Preparation 3 (P3): 2-(2-Methoxy-5-nitro-phenoxy)-1-(4-methyl-piperidin-1-yl)-ethanone

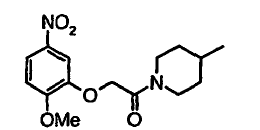

To a NaOH solution (14 g of NaOH, 350 mmol, in 300 ml of water) at 0 °C containing 4-metylpiperidine (36 ml, 310 mmol) chloroacetylchloride (23.7 ml, 305 mmol) was added in 10 min under vigorous stirring at 0 °C. After 3 h the mixture was acidified using HCl (2M aqueous) and the acqueous phase was extracted twice with CH₂Cl₂ (2 x 150 ml). The combined organic extracts were dried over anhydrous Na₂SO₄, filtered and concentrated to dryness *in vacuo* to give 37 g of intermediate which was dissolved in *N*-methyl pyrrolidone (120 ml).
To this solution 2-methoxy-5-nitrophenol (24 g, 156 mmol), K₂CO₃ (21 g, 152 mmol) and Nal (2 g, 13 mmol) were added. The suspension was mechanically stirred and heated at 120 °C. After 16 h the reaction was cooled and diluted with Et₂O (500 ml), the organic phase was washed twice with water (2x 150 ml), then with NaOH (aqueous, 1 M, 100 ml) and finally with brine (150 ml).
The organic phase was dried over anhydrous Na₂SO₄, filtered and concentrated to dryness *in vacuo* to give 35 g of the title compound as a yellow solid. **NMR** (¹H, **CDCl₃):** δ 8.00 (dd, 1H), 7.76 (d, 1H), 6.98 (d, 1H), 4.87 (s, 2H), 4.55 (d, 1H), 4.02 (s, 3H), 3.85 (d, 1H), 3.13 (t, 1H), 2.68 (t, 1H), 1.55-1.85 (m, 4H), 1.10-1.30 (m, 1H), 1.01 (d, 3H). **MS (m/z):** 309 [MH]⁺.

### Preparation 4 (P4): 1-[2-(2-Methoxy-5-nitro-phenoxy)-ethyl]-4-methyl-piperidine

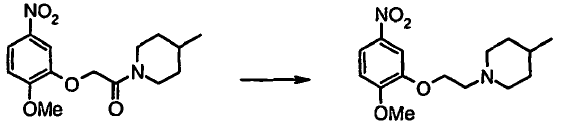

2-(2-Methoxy-5-nitro-phenoxy)-1-(4-methyl-piperidin-1-yl)-ethanone (11 g, 35.7 mmol) was dissolved in anh. THF (20 ml), 1M BH₃.THF (2.2eq, 78.5 ml) was added dropwise to the solution, and the mixture was heated at reflux for 4 hours. The solution was cooled to room temperature, CH₃OH (100 ml) was added, and the solvent was removed under reduced pressure. The residue was dissolved in CH₃OH (100 ml), 6N HCl (200 ml) was added, and the solution was heated to reflux for 30'. The CH₃OH was removed under reduced pressure and the remaining aqueous solution was made basic (pH>10) with 2.5 M NaOH. The basic solution was extracted with ethyl acetate (3x80 ml). The combined organic extracts were dried over anhydrous Na₂SO₄ and the solvent was removed under reduced pressure to give 9.4 g of the title product as a red oil. (yield: 90%). **NMR (¹H, CDCl₃):** δ 7.91 (dd, 1H), 7.80 (d, 1H), 6.90 (d, 1H), 4.21 (t, 2H), 3.96 (s, 3H), 2.97 (m, 2H), 2.85 (t, 2H), 2.12 (m, 2H), 1.64 (m, 2H), 1.4 (m, 1H), 1.29 (m, 2H), 0.92 (d, 3H). **MS (*m*/*z*)**: 295 [MH]⁺.

### Preparation 5 (P5): 4-Methoxy-3-[2-(4-methyl-piperidin-1-yl)-ethoxy]-phenylamine (hydrochloride)

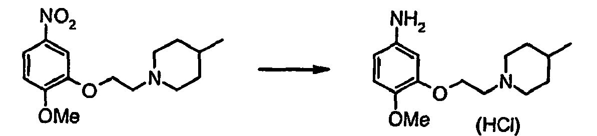

To a solution of 1-[2-(2-methoxy-5-nitro-phenoxy)-ethyl]-4-methyl-piperidine (9.5 g, 32.3 mmol) in abs. EtOH (200 ml) were added Pd/C 10% (1 g) and the reaction mixture was hydrogenated at room temperaturature at 1 atm. After 5 hrs the reaction mixture was filtered on a Celite pad and the solution was concentrated *in vacuo* to give 8.5 g of the title product as a brown solid (yield: 99%). **NMR (¹H, CDCl₃):** δ 6.61 (d, 1H), 6.26 (d, 1H), 6.04 (dd, 1H), 4.60 (bs, 2H), 3.91 (t, 2H), 3.58 (s, 3H), 2.85 (m, 2H), 2.61 (t, 2H), 1.97 (m, 2H), 1.52 (m, 2H), 1.3 (m, 1H), 1.1 (m, 2H), 0.86 (d, 3H).
Batches of this material were converted to the hydrochloride salt as required as described for Preparation 2.

### Preparation 6 (P6): 2-Bromo-6-methyl-benzoic acid methyl ester

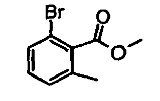

The title compound was obtained from 2-bromo-6-methyl-benzoic acid according to Procedure G3 (quant. yield): **NMR (¹H, CDCl₃):** δ 7.47 (t, 1H), 7.19-7.23 (m, 2H), 4.03 (s, 3H), 2.40 (s, 3H).

The compounds from Preparations 7 and 8 were prepared by applying Procedure G2 to the respective substituted toluenes:

| | | |
|---|---|---|
| **Preparation 7 (P7): 2-Bromomethyl-5-fluoro-benzoic acid methyl ester** | 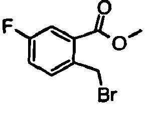 | **NMR (¹H, CDCl₃):** δ 7.63 (dd, 1H), 7.40-7.45 (m, 1H), 7.13-7.18 (m, 1H), 4.90 (s, 2H), 3.93 (s, 3H). |
| **Preparation 8 (P8): 2-Bromo-6-bromomethyl-benzoic acid methyl ester** | 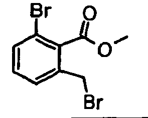 | **NMR (¹H, CDCl₃):** δ 7.52 (d, 1H), 7.41 (d. 1H), 7.18-7.27 (m, 1H), 4.46 (s, 2H), 3.95 (s, 3H). |

### Preparation 9 (P9): 7-Chloro-3-methyl-3H-isobenzofuran-1-one:

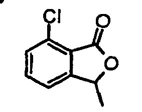

To 2-chlorobenzoic acid (0.32 g) in dry THF (10 ml) was added slowly via syringe with vigorous stirring at -78 °C *sec*-butyllithium (1.3 M in cyclohexane, 3.7 ml). After 1 h at -78 °C acetaldehyde (0.17 ml) was added in one portion with vigorous stirring. The mixture was allowed to warm to 0 °C before conc. aqueous HCl (0.4 ml) was added. The mixture was concentrated *in vacuo,* partitioned between water and CH₂Cl₂, the organic layer concentrated and submitted to column chromatography to yield the title compound as a colourless waxy solid (0.11 g). **NMR (¹H, CDCl₃):** δ 7.55 (t, 1H), 7.43 (d, 1H), 7.30 (d, 1H), 5.47 (q, 1H), 1.60 (d, 3H). **MS (*m*/z)*:*** 183 [MH]⁺ (1CI).

### Preparation 10 (P10): 3,4-Difluoro-phtalic acid-2-methyl ester

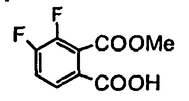

To a solution of 3,4-difluorophthalic anhydride (200 mg, 1.09 mmol) in dry MeOH (2 ml) at 0 °C was added MeONa (30% in MeOH, 1.14mmol). The solution was allowed to warm to room temperature and stirred for 3 hours. After removing the solvent under reduced pressure, the residue was diluted with water, acidified using conc. aqueous HCl (1.09 mmol) and extracted with EtOAc (x3). The organic phases were collected, dried over Na₂SO₄, filtered and concentrated to dryness *in vacuo* to give the title compound (186 mg). **NMR (¹H, d6-DMSO):** δ 13.6 (bs, 1H), 7.88/7.75 (m, 2H), 3.80 (s, 3H). **MS (m/z):** 215 [M-H].

### Preparation 11 (P11): 6,7-Difluorophthalide

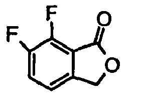

3,4-Difluoro-phthalic acid-2-methyl ester (185 mg, 0.86 mmol), triethylamine (0.86 mmol) and ethyl chloroformate (0.86 mmol) were dissolved in 2 ml of dry THF under stirring at 0 °C. The mixture was stirred for an additional 30 minutes at 0 °C then the precipitate was filtered off and washed with THF. The filtrate was added dropwise to a solution of NaBH₄ (2.14 mmol) in H₂O (1 ml) at 0 °C. When the addition was completed the mixture was stirred at room temperature for 1 hour. The mixture was acidified with 1N HCl and concentrated under reduced pressure and the residue was extracted with CH₂Cl₂. The extract was dried over Na₂SO₄ and evaporated to give the title compound (100 mg). **NMR (¹H, d6-DMSO):** δ 7.97 (dd, 1H), 7.80 (dd, 1H), 5.37 (s, 2H).

### Preparation 12 (P12): 4,5-Dichloro-phthalic acid-2-methyl ester

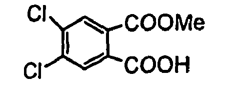

To a solution of 4,5-dichlorophthalic anhydride (5.0 g, 23 mmol) in dry MeOH (60 ml) at 0 °C was added MeONa (30% in MeOH, 24.2 mmol). The solution was allowed to warm to room temperature and stirred for 3 hours. After removing the solvent under reduced pressure, the residue was diluted with water, acidified using conc. aqueous HCl (23 mmol) and extracted with EtOAc (x3). The organic phases were collected, dried over Na₂SO₄, filtered and concentrated to dryness *in vacuo* to give the title compound as a colourless solid (5.3 g): **NMR (¹H, d6-DMSO)**: δ 13.7 (bs, 1H), 8.00 (s, 1H), 7.95 (s, 1H), 3.80 (s, 3H). **MS (*m*/*z*)**: 247 [M-H]⁻ (2Cl).

### Preparation 13 (P13): 5,6-Dichlorophthalide

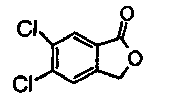

To 4,5-dichlorophthalic acid-2-methyl ester (22.7 mmol) and triethylamine (3.5 ml) in dry THF (75 ml) was added over 10 min *via* syringe at 0 °C with vigorous stirring ethyl chloroformate (2.4 ml). The mixture was stirred for an additional 30 minutes at 0 °C. The precipitate was removed by filtration and washed with THF (10 ml). The filtrate was added over 15 min dropwise with stirring to a solution of NaBH₄ (2.2 g) in H₂O:THF 1:1 (75 ml) at 0 °C. The mixture was stirred at room temperature for 1 hour, acidified (1 M aqueous HCl, 100 ml) and partially concentrated under reduced pressure (to remove the bulk THF). The product was collected by filtration, washed with water and a small volume of MeOH (2 ml). The material thus obtained was partitioned between sat. aqueous NaHCO₃ and CH₂Cl₂, dried (MgSO₄) and evaporated to give the title compound (3.2 g) as a colourless solid:
**NMR (¹H, d6-DMSO):** δ 8.12 (s, 1H), 8.00 (s, 1H), 5.37 (s, 2H).

### Preparation 14 (P14): 5,7-Dichloro-2-(3-hydroxy-4-methoxy-phenyl)-2,3-dihydroisoindol-1-one

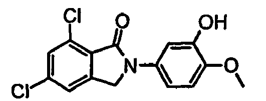

To 2,4-dichlorobenzoic acid (2.76 g) in dry THF (100 ml) was added slowly via syringe with vigorous stirring at -78 °C *sec*-butyllithium (1.4 M in cyclohexane, 23.7 ml). After 1 h at -78 °C methyl iodide (1.17 ml) was added in one portion with vigorous stirring. The mixture was allowed to warm to 25 °C over 4 h and water was added. After 5 min it was partitioned between aqueous HCl (1M) and EtOAc. The organic layer was washed (brine), dried (MgSO₄), filtered and concentrated *in vacuo* to give a yellow oil (3 g) that was treated as described in procedure G3 (assuming 14.4 mmol substituted benzoic acid content). Column chromatography gave a yellow oil (3.0 g) which consisted of a mixture of compounds as judged by NMR. This material (using 3 g *N*-bromosuccinimide) was treated as described in procedure G2 to give , besides 3-bromomethyl-2,4-dichloro benzoic acid methyl ester (1.1 g) a colourless oil (1.9 g) consisting of a mixture of compounds as judged by NMR, containing 2-bromomethyl-4,6-dichlorobenzoic acid methyl ester. This material (estimated from NMR to contain 2.9 mmol substituted benzyl bromide) and 5-amino-2-methoxy phenol (0.48 g) in dry DMF (2 ml) were heated in a microwave reactor to 150 °C for 12 min. The mixture was concentrated *in vacuo,* triturated with CH₂Cl-₂:EtOAc:petroleum ether 1:1:5 (ca. 15 ml), and then ca. 2 ml each of CH₂Cl₂ and MeOH to give the title compound as an off-white solid (0.61 g). Additional material 0.06 g) was obtained from chromatography of the organic extracts: **NMR (¹H, d6-DMSO):** δ 9.16 (s, 1H), 7.76-83 (m, 2H), 7.37 (s, 1H), 7.12 (d, 1H), 6.95 (d, 1H), 4.88 (s, 2H), 3.75 (s, 3H). **MS (*m*/*z*):** 322 [M-H]⁻ (2Cl).

### Preparation 15 (P15): 2,4-Dichloro-3-methyl benzoic acid methyl ester

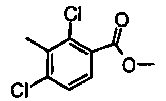

3-Bromomethyl-2,4-dichloro benzoic acid methyl ester (1.1 g, cf. synthesis in P14) was stirred for 1 h at 25 °C with Zn powder (0.5 g) in HOAc (5 ml). Et₂O (20 ml) was added, the mixture filtered, washing with Et₂O. The solution was concentrated *in vacuo* and the residue submitted to column chromatography to give product as a slowly solidifying colourless oil (0.54 g): **NMR (¹H, CDCl₃):** δ 7.50 (d, 1H), 7.31 (d, 1H), 3.90 (s, 3H), 2.50 (s,3H).

### Preparation 16 (P16): 2,4-Dichloro-3-methyl benzoic acid

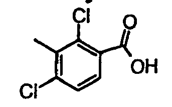

2,4-Dichloro-3-methyl benzoic acid methyl ester was heated at 45 °C for 1 h in MeOH (5 ml), THF (5 ml) and aqueous KOH (2 M, 2.5 ml). Water was added, the mixture concentrated somewhat *in vacuo*, acidified (aqueous HCl 1 M) and extracted with EtOAc. The organic layer was washed (brine), dried (MgSO₄), concentrated and triturated with Et₂O : petroleum ether 1:1 to give the title compound as a colourless solid (0.43 g): **NMR (¹H, CDCl₃):** δ 7.70 (d, 1H), 7.35 (d, 1H), 2.52 (s,3H) (carboxylic acid proton not observed). **MS (*m*/*z*):** 203 [M-H]⁻ (2Cl).

### Preparation 17 (P17): 5,7-Dichloro-6-methyl-phthalide

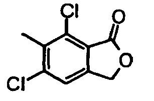

To 2,4-dichloro-3-methyl benzoic acid (0.43 g) in dry THF (10 ml) was added slowly via syringe with vigorous stirring at -78 °C *sec*-butyllithium (1.4 M in cyclohexane, 3.5 ml). After 1 h formaldehyde (generated by thermal depolymerisation of 0.4 g paraformaldehyde and transferred *via* a nitrogen stream through a canula) was blown onto the reaction mixture with vigorous stirring. The mixture was allowed to warm to 0 °C over 90 min and conc. aqueous HCl (1 ml) was added. After 1 h at 25°C the mixture was concentrated *in vacuo* and submitted to column chromatography to give the title compound as a colourless creamy solid (0.14 g): **NMR (¹H, CDCl₃): δ** 7.41 (s, 1H), 5.18 (s, 2H), 2.54 (s, 3H).

### Preparation 18 (P18): 2-(tert-Butyl-dimethyl-silanyloxymethyl)-4,5-dichloro-benzoic acid

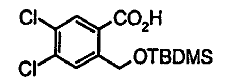

5,6-Dichlorophthalide (P13, 1.5 g) was heated at 50 °C for 2 h in MeOH (15 ml), THF (15 ml) and aqueous KOH (2 M, 7.5 ml). Aqueous HCl (1 eq., 2 M) was added, the mixture concentrated to dryness *in vacuo,* and triturated with THF and subsequently with iPrOH to give a colourless solid (2 g). To this material in DMF (dry, 10 ml) and DCM (dry, 10 ml) was added imidazole (0.75 g) and *tert*-butyldimethylsilyl chloride (TBDMS-Cl, 1.7 g). After 16 h at 25 °C excess water was added, the mixture concentrated somewhat, acidified (1 M aqueous HCl) and extracted with Et₂O. The organic layer was washed (water, 2 * brine), dried (MgSO₄), concentrated, triturated with some petroleum ether and submitted to column chromatography to give the title compound as a colourless solid (0.29 g, additional product contained in the petroleum ether solution from the trituration): **NMR (¹H, CDCl₃):** δ 8.13 (s, 1H), 7.87 (s, 1H), 5.00 (s, 2H), 0.94 (s, 9H), 0.12 (s, 6H) (carboxylic acid proton not observed). **MS (*m*/*z*)*:*** 333 [M-H]⁻ (2Cl).

### Preparation 19 (P19): 5,6-Dichloro-7-methyl-phthalide

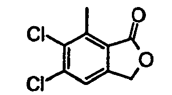

To 2-(*tert*-butyl-dimethyl-silanyloxymethyl)-4,5-dichloro-benzoic acid (0.28 g) in dry THF (5 ml) was added slowly via syringe with vigorous stirring at -78 °C *sec*-butyllithium (1.4 M in cyclohexane, 1.4 ml). After 1 h at -78 °C methyl iodide (0.078 ml) was added in one portion with vigorous stirring. The mixture was allowed to warm to 25 °C over 4 h and water and excess conc. aqueous HCl were added. The mixture was concentrated *in vacuo* and treated with excess HCl in Et₂O and AcOEt for 1 h. The mixture was concentrated *in vacuo* and submitted to column chromatography to give the title compound as an orange solid (0.13 g): **NMR (¹H, CDCl₃):** δ 7.42 (s, 1H), 5.18 (s, 2H), 2.80 (s, 3H).

### Preparation 20: 2-(2-Methoxy-5-nitro-phenoxy)-ethanol

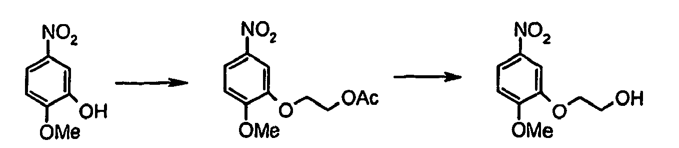

To a solution of 2-methoxy-5-nitro-phenol (1.0 g, 5.9 mmol) in dry DMF (20 ml), under N₂, was added K₂CO₃ (1.1 eq., 0.9 g) and 2-bromoethyl acetate (1.1 eq, 0.714 ml). After heating to 60 °C for 4 hours, a saturated solution of NH₄Cl was added and the mixture was extracted with dichloromethane. The organic phase was washed with water, dried over Na₂SO₄, filtered and then concentrated *in vacuo,* to give 1.5 g of the crude intermediate compound. This was dissolved in dry methanol (34 ml) and MeONa (31 mg, 0.58 mmol) was added. After 1 h an excess of Amberlite IR120 (H⁺ form) was added, the solution was filtered and concentrated in vacuo. The residue was dissolved in dichloromethane and washed with NaOH 0.1 N and water. The organic phase was dried over Na₂SO₄, filtered and then concentrated *in vacuo,* to give 1.1 g of the title compound as a pale yellow solid (m.p. 115 °C). **NMR (¹H, CDCl₃):** δ 7.95 (dd, 1H), 7.80 (d, 1H), 6.94 (d, 1H), 4.21 (t, 2H), 4.03 (t, 2H), 3.98 (s, 3H), 2.3 (bs, 1H). **MS (*m*/*z*):** 214 [MH]⁺.

### Preparation 21: 1-[2-(2-Methoxy-5-nitrophenoxy)ethyl]-4,4-dimethylpiperidine-2,6-dione

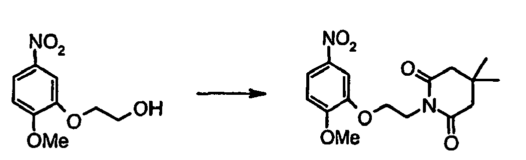

Crude 2-(2-methoxy-5-nitro-phenoxy)-ethanol (0.43 g, 2 mmol) was dissolved in dry THF, 4,4-dimethylglutarimide (0.28 g, 2 mmol), triphenylphosphine (0.52 g, 2 mmol) and diethylazodicarboxylate (1.1 ml, 2.4 mmol) were added at 45 °C. After 6 h the reaction mixture was concentrated *in vacuo* and purified by flash-chromatography (cyclohexane/ ethyl ether 3/7) to give 0.41 g of the title compound as a white solid. **NMR (¹H, CDCl₃)**: δ 7.88 (d, 1H), 7.73 (s, 1H), 6.85 (d, 1H), 4.30-4.15 (m, 4H), 3.90 (s, 3H), 2.52 (s, 4H), 1.13 (s, 6H).

### Preparation 22: 1-[2-(2-Methoxy-5-nitro-phenoxy)ethyl]-4,4-dimethylpiperidine

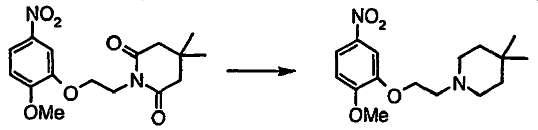

1-[2-(2-Methoxy-5-nitrophenoxy)ethyl]-4,4-dimethylpiperidine-2,6-dione (426 mg, 1.26 mmol) was dissolved in dry THF (13 ml), 1M BH₃.THF (2.2 eq, 3.8 ml) was added dropwise to the solution, and the mixture was heated at reflux for 4 hours. The solution was cooled to room temperature, CH₃OH (10 ml) was added, and the solvent was removed under reduced pressure. The residue was dissolved in CH₃OH (10 ml), 6N HCl (10 ml) was added, and the solution was heated to reflux for 2 h. The CH₃OH was removed under reduced pressure and the remaining aqueous solution was made basic (pH>10) with 2.5 M NaOH. The basic solution was extracted with ethyl acetate (3x20 ml). The combined organic extracts were dried over anhydrous Na₂SO₄ and the solvent was removed under reduced pressure to give after flash chomatography (cyclohexane/ethyl acetate 3/7) the title compound (240 mg) as a yellow oil (62%): **NMR (¹H, CDCl₃):** δ 7.85 (d, 1H), 7.75 (s, 1H), 6.85 (d, 1H), 4.17 (t, 2H), 3.92 (s, 3H), 2.84 (t, 2H), 2.46 (m, 4H), 1.37 (m, 4H), 0.86 (s, 6H). **MS (*m*/*z*):** 309 [MH]⁺.

### Preparation 23: 3-[2-(4,4-Dimethylpiperidin-1-yl)ethoxy]-4-methoxyphenylamine

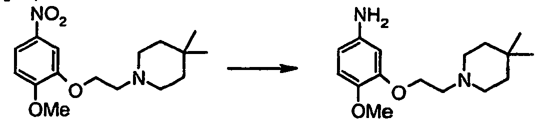

1-[2-(2-Methoxy-5-nitro-phenoxy)ethyl]-4,4-dimethylpiperidine (337 mg) was dissolved in MeOH (12 ml), Pd/C 10% was added and the mixture was allowed to react under H₂ at atmospheric pressure for 3 h. The mixture was filtered and concentrated *in vacuo* to give 328 mg of the title compound as a pink oil: **NMR (¹H, CDCl₃):** δ 6.67 (d, 1H), 6.30 (s, 1H), 6.22 (d, 1H), 4.12 (t, 2H), 3.76 (s, 3H), 2.78 (t, 2H), 2.46 (m, 4H), 1.37 (m, 4H), 0.86 (s, 6H) (NH₂ not observed). **MS (*m*/*z*)*:*** 279 [MH]⁺.

### Preparation 24: 1-[2-(2-Methoxy-5-nitrophenoxy)ethyl]azepane

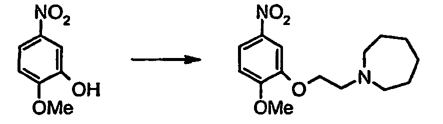

2-Methoxy-4-nitro-phenol (338 mg) was allowed to react with 2-(hexamethylene-imino)ethyl chloride hydrochloride following Preparation 20 to give 417 mg of the title compound as a red oil: **NMR (¹H, CDCl₃):** δ 7.92 (dd, 1H), 7.75 (d, 1H), 6.87 (d, 1H), 4.28 (t, 2H), 3.95 (s, 3H), 3.05 (t, 2H), 2.78 (m, 4H), 1.80-1.45 (m, 8H). **MS (*m*/*z*)*:*** 295 [MH]⁺.

### Preparation 25: 3-(2-Azepan-1-ylethoxy)-4-methoxyphenylamine

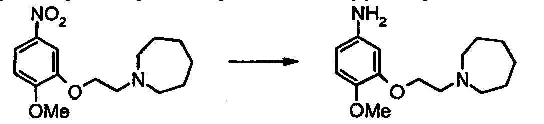

1-[2-(2-Methoxy-5-nitrophenoxy)ethyl]azepane (324 mg) was reduced following Preparation 23 to give the title compound (230 mg) as a red oil: **NMR (¹H, CDCl₃)**: δ 6.68 (d, 1H), 6.35 (d, 1H), 6.20 (d, 1H), 4.15 (t, 2H), 3.75 (s, 3H), 3.4-3.6 (m, 2H), 2.98 (t, 2H), 2.80 (m, 4H), 1.78-1.42 (m, 8H). **MS (*mlz*)**: 265 [MH]⁺.

### Example 1: 2-[4-Methoxy-3-(2-piperidin-1-yl-ethoxy)phenyl]-2,3-dihydroisoindol-1-one hydrochloride

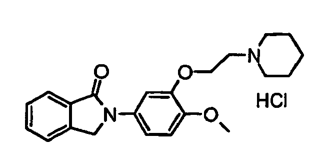

Benzyl 2-(chloromethyl)benzoate [obtained from the reaction of 2-(chloromethyl) benzoyl chloride (e.g. Hinton, I.G., Mann, F.G. *J. Chem. Soc*. **1959**, 599-610) with benzyl alcohol] and 4-methoxy-3-(2-piperidin-1-yl-ethoxy)-phenylamine hydrochloride (P2, 1 eq.) in dry DMF (0.5 ml) were heated to 120 °C for 1 h. The mixture was concentrated *in vacuo* and submitted to column chromatography, follwed by conversion to the hydrochloride salt (G1) to give the title compound as an off-white waxy solid (30 mg): **NMR (¹H, d6-DMSO):** δ 10 (t, 1H), 7.75-7.77 (m, 2H), 7.65-7.69 (m, 2H), 7.53-7.56 (m, 1H), 7.37 (dd, 1H), 7.10 (d, 1H), 5.00 (s, 2H), 4.39 (t, 2H), 3.80 (s, 3H), 3.57 (bd, 2H), 3.48-3.51 (m, 2H), 3.00-3.06 (m, 2H), 1.68-1.84 (m, 5H), 1.37-1.41 (m, 1H).
**MS *(mlz):*** 367 [MH]⁺.

### Example 2: 6-Fluoro-2-[4-methoxy-3-(2-piperldin-1-yl-ethoxy)phenyl]-2,3-dihydroisoindol-1-one hydrochloride

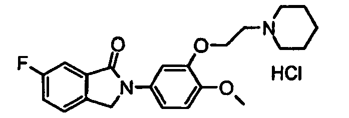

2-Bromomethyl-5-fluoro-benzoic acid methyl ester (P7, 0.12 g) and 4-methoxy-3-(2-piperidin-1-yl-ethoxy)-phenylamine hydrochloride (P2, 1.05 eq.) in dry DMF (0.5 ml) were heated to 120 °C for 2 h. The mixture was concentrated *in vacuo,* the residues partitioned between aqueous NaHCO₃ and EtOAc, the organic layer concentrated and submitted to column chromatography, follwed by conversion to the hydrochloride salt (G1) to give the title compound as an off-white solid (0.18 g): **NMR (¹H, CDCl₃):** δ 7.42-7.62 (m, 3H), 7.36 (d, 1H), 7.25-7.30 (m, 1H), 6.90 (d, 1H), 4.82 (s, 2H), 4.61 (bs, 2H), 3.85 (s, 3H), 3.61-3.74 (m, 2H), 3.37-3.47 (m, 2H), 2.83-2.97 (m, 2H), 2.18-2.38 (m, 2H), 1.80-1.95 (m, 3H), 1.35-1.51 (m, 1H) (hydrochloride proton not observed). **MS *(m*/*****z)**:* 385 [MH]⁺.

### Example 3: 7-Bromo-2-{4-methoxy-3-[2-(4-methyl-piperidin-1-yl)-ethoxy]phenyl}-2,3-dihydroisoindol-1-one hydrochloride

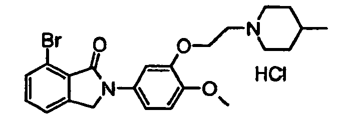

2-Bromo-6-bromomethyl-benzoic acid methyl ester (P8, 0.71 mmol) and 4-methoxy-3-[2-(4-methyl-piperidin-1-yl)-ethoxy]-phenylamine hydrochloride (P5, 1.5 eq.) in dry DMF (2 ml) were heated in a microwave reactor to 150 °C for 10 min. The mixture was concentrated *in vacuo,* and the residue submitted to column chromatography, follwed by conversion to the hydrochloride salt (G1) to give the title compound as a colourless powder: **NMR (¹H, d4-MeOH):** δ 7.79 (d, 1H), 7.69 (d, 1H), 7.61 (d, 1H), 7.52 (t, 1H), 7.26 (dd, 1H), 7.12 (d, 1H), 4.91 (s, 2H), 4.41 (t, 2H), 3.90 (s, 3H), 3.73-3.79 (m, 2H), 3.58 (bs, 2H), 3.11 (bt, 2H), 1.93-2.02 (m, 2H), 1.66-1.82 (m, 1H), 1.43-1.58 (m, 2H), 1.04 (d, 3H)(only data for major protonation isomer given). **MS** *(mlz):* 459/461 [MH]⁺ (1 Br).

### Example 4: 7-Chloro-2-{4-methoxy-3-[2-(4-methyl-piperidin-1-yl)-ethoxy]phenyl}-3-methyl-2,3-dihydroisoindol-1-one hydrochloride

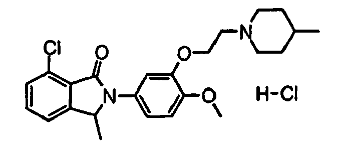

To 4-methoxy-3-[2-(4-methyl-piperidin-1-yl)-ethoxy]-phenylamine (P5, 0.47 mmol) in dry toluene (1 ml) was added trimethylaluminium (2 M solution in hexanes, 1.1 ml). After 15 min a solution of 7-chloro-3-methyl-3*H*-isobenzofuran-1-one (P9) in dry CH₂Cl₂ (5 ml) was added. After 20 h the mixture was heated at 37 °C for 5 h. After cooling silica gel and CH₂Cl₂ were added and the material filtered and washed with a 1:10:30 mixture of conc. aqueous NH₃:MeOH:CH₂Cl₂ . Column chromatography of the resulting solution after concentration gave a brown film (0.12 g) which consisted of a mixture of compounds as judged by NMR. To this material was added PPh₃ (0.26 g) and THF (dry, 1 ml) followed by slow addition over 20 min at 50 °C of diisoropyl azodicarboxylate (0.20 ml). After 30 min volatiles were evaporated and the residue submitted to repeated column chromatography followed by salt formation (G1) to give the title compound as a colourless film (12 mg). **NMR (¹H, d4-MeOH):** δ 7.57-7.65 (m, 2H), 7.50 (dd, 1H), 7.36 (d, 1H), 7.12-7.14 (m, 2H), 5.28 (q, 1H), 4.40 (q, 2H), 3.92 (s, 3H), 3.75 (d, 2H), 3.55 (t, 2H), 3.10 (t, 2H), 1.98 (d, 2H), 1.74 (m, 1H), 1.50 (d, 2H), 1.40 (d, 3H), 1.04 (d, 3H)(only data for major protonation isomer given). **MS (*m*/*z*):** 429 [MH]⁺ (1Cl).

### Example 5: 2-{4-Methoxy-3-[2-(4-methyl-piperidin-1-yl)-ethoxy]phenyl}-7-trifluoromethyl-2,3-dihydroisoindol-1-one hydrochloride

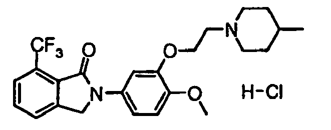

To 2-trifluoromethylbenzoic acid (0.95 g) in dry THF (30 ml) was added slowly via syringe with vigorous stirring at -78 °C *sec*-butyllithium (1.3 M in cyclohexane, 9.2 ml). After 1 h at -78 °C methyl iodide (0.75 ml) was added in one portion with vigorous stirring. The mixture was allowed to slowly warm to 25 °C and kept for 16 h before sat. aqueous NaHCO₃ was added. The mixture was stirred for 3 h, mixed with EtOAc:petroleum ether 1:1 and the layers were separated. The aqueous layer was acidified using 2 M aqueous HCl and extracted with EtOAc. This organic layer was collected, concentrated *in vacuo* and treated as described in procedure G3. Column chromatography gave a colourless oil (0.83 g) which consisted of a mixture of compounds as judged by NMR. This material (assuming 2 mmol content of the substituted toluene) was treated as described in procedure G2 to give material (0.73 g) consisting of a mixture of compounds as judged by NMR. This material and 4-methoxy-3-[2-(4-methyl-piperidin-1-yl)-ethoxy]-phenylamine hydrochloride (P5, 2.3 mmol) in dry DMF (4.5 ml) were heated in a microwave reactor to 150 °C for 15 min. The mixture was concentrated *in vacuo*, and the residue submitted to column chromatography, follwed by conversion to the hydrochloride salt (G1) to give the title compound as a faint yellow solid: **NMR (¹H, d4-MeOH):** δ 7.89 (t, 1H), 7.85-7.80 (m, 3H), 7.26 (dd, 1H), 7.12 (d, 1H), 5.01 (s, 2H), 4.42 (t, 2H), 3.90 (s, 3H), 3.76 (d, 2H), 3.58 (t, 2H), 3.12 (t, 2H), 1.98 (d, 2H), 1.74 (m, 1H), 1.50 (q, 2H), 1.04 (d, 3H)(only data for major protonation isomer given). **MS (*m*/*z*):** 449 [MH]⁺.

### Example 6: 5,7-Dichloro-2-{4-methoxy-3-[2-(4-methyl-piperidin-1-yl)-ethoxy]phenyl}-2,3-dihydroisoindol-1-one hydrochloride

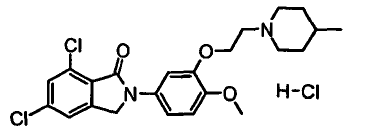

To 2,4-dichlorobenzoic acid (0.96 g) in dry THF (30 ml) was added slowly via syringe with vigorous stirring at -78 °C sec-butyllithium (1.3 M in cyclohexane, 9.2 ml). After 1 h at -78 °C methyl iodide (0.44 ml) was added in one portion with vigorous stirring. The mixture was allowed to warm to 25 °C over 4 h and water was added. After 5 min it was partitioned between aqueous HCl (1 M) and EtOAc. The organic layer was washed (brine), dried (MgSO₄), filtered and concentrated *in vacuo* to give a yellow oil that was treated as described in procedure G3 (assuming 5 mmol substituted benzoic acid content). Column chromatography gave a yellow oil (0.81 g) which consisted of a mixture of compounds as judged by NMR. This material (assuming 3.7 mmol content of the of substituted toluene) was treated as described in procedure G2 to give a colourless oil (0.59 g) consisting of a mixture of compounds as judged by NMR. Half of this material and 4-methoxy-3-[2-(4-methyl-piperidin-1-yl)-ethoxy]-phenylamine hydrochloride (P5, 0.65 mmol) in dry DMF (1.3 ml) were heated in a microwave reactor to 150 °C for 15 min. The mixture was concentrated *in vacuo,* and the residue submitted to column chromatography, follwed by conversion to the hydrochloride salt (G1) to give the title compound as a faint yellow solid (0.11 g): **NMR (¹H, d4-MeOH):** δ 7.76 (d, 1H), 7.62 (s, 1H), 7.57 (s, 1H), 7.24 (dd, 1H), 7.11 (d, 1H), 4.91 (s, 2H), 4.40 (t, 2H), 3.90 (s, 3H), 3.72-3.79 (m, 2H), 3.57 (t, 2H), 3.11 (t, 2H), 1.90-2.04 (m, 2H), 1.65-1.82 (m, 1H), 1.44-1.57 (m, 2H), 1.04 (d, 3H) (only data for major protonation isomer given). **MS (*m*/*z*):** 449 [MH]⁺ (2Cl).

### Example 7: 7-Chloro-2-[4-methoxy-3-(2-piperidin-1-yl-ethoxy)phenyl]-2,3-dihydroisoindol-1-one hydrochloride

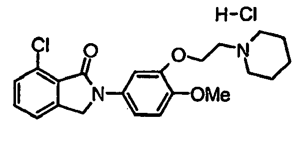

2-Chloro-6-methylbenzoic acid (887 mg) was treated as described in procedure G3 to give the corresponding ester (929 mg).
Methyl 2-chloro-6-methyl benzoate (185 mg) was treated as described in procedure G2 to give the respective benzyl bromide as a colourless oil (213 mg) after flash chromatography (ciclohexane/ethyl acetate 95/5).
After heating to 60 °C for 2h the bromide, 4-methoxy-3-(2-piperidin-1-yl-ethoxy)-phenylamine (P2, 0.1 mmol) and K₂CO₃ (0.1 mmol) in dry DMF (5 ml) and 1,4-dioxane (3 ml), 2,6-lutidine (0.76 mmol) was added. The mixture was heated to 95 °C for 4h, then a saturated solution of NH₄Cl was added, the mixture was extracted with dichloromethane, washed with brine and concentrated *in vacuo.* The residue was submitted to column chromatography (eluent: CH₂Cl₂/MeOH = 95/5), the free base was then converted to the hydrochloride salt (G1), to give a light brown powder (70 mg). **NMR (¹H, CDCl₃):** δ 9.43 (bs, 1H), 7.75 (d, 1H), 7.65 (m, 2H), 7.53 (d, 1H), 7.32 (dd, 1H), 7.10 (d, 1H), 4.97 (s, 2H), 4.38 (t, 2H), 3.81 (s, 3H), 3.62 (d, 2H), 3.49 (t, 2H), 3.05 (t, 2H), 1.81 (m, 4H), 1.6 (m, 2H). **MS (*m*/*z*):** 401 [MH]⁺ (1Cl).

### Example 8: 6-Chloro-2-[4-methoxy-3-(2-piperidin-1-yl-ethoxy)phenyl]-2,3-dihydroisoindol-1-one hydrochloride

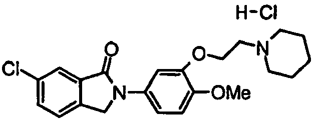

The title compound was prepared in an analogous manner to the compound of Example 7. **NMR (¹H, CDCl₃):** δ 10.15 (bs, 1H), 7.72 (m, 4H), 7.36 (dd, 1H), 7.12 (d, 1H), 5.0 (s, 2H), 4.41 (t, 2H), 3.81 (s, 3H), 3.55 (m, 2H), 3.49 (t, 2H), 3.05 (m, 2H), 1.81 (m, 4H), 1.4 (m, 2H). **MS (*m*/*z*):** 401 [MH]⁺ (1 Cl).

### Example 9: 5-Chloro-2-[4-methoxy-3-(2-piperidin-1-yl-ethoxy)phenyl]-2,3-dihydroisoindol-1-one hydrochloride

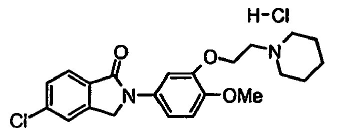

The title compound was prepared in an analogous manner to the compound of Example 7. **NMR (¹H, CDCl₃): δ** 9.84 (bs, 1H), 7.75 (d, 1H), 7.70 (d, 1H), 7.67 (d, 1H), 7.55 (dd, 1H), 7.31 (dd, 1H), 7.06 (d, 1H), 4.95 (s, 2H), 4.33 (t, 2H), 3.76 (s, 3H), 3.52 (m, 2H), 3.46 (m, 2H), 3.0 (m, 2H), 1.72 (m, 4H), 1.35 (m, 2H). **MS *(m*/*****z):*** 401 [MH]⁺ (1Cl).

### Example 10: 5,7-Dichloro-2-{4-methoxy-3-[2-(cis-2,6-dimethyl-piperidin-1-yl)-ethoxy]phenyl}-2,3-dihydroisoindol-1-one hydrochloride

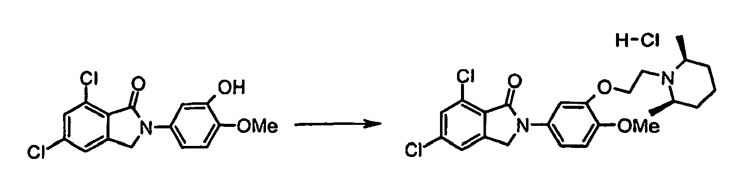

To a solution of *cis*-2,6-dimethylpiperidine (1.48 mmol) and triethylamine (1.78 mmol) in dichloromethane (15 ml) was added chloroacetylchloride (1.78 mmol) at 0 °C. After 5h at 25 °C the mixture was washed with 5% NaHCO₃, brine and evaporated *in vacuo* to give a yellow solid (433 mg). The α-cloroamide (200 mg) was reduced as described in procedure P4 to give 1-(2-chloroethyl)-2,6-dimethy-piperidine (139 mg).
To a solution of 5,7-dichloro-2-(3-hydroxy-4-methoxy-phenyl)-2,3-dihydroisoindol-I -one (P14, 78 mg, 0.24 mmol) in DMF (1.5 ml) at 25 °C were added K₂CO₃ (40 mg, 0.29mmol) and 1-(2-chloroethyl)-2,6-dimethy-piperidine (51 mg, 0.29 mmol). The suspension was stirred at 40 °C for 6h, then at room temperature for 72 h. The suspension was diluted with a saturated solution of NH₄Cl and extracted with dichloromethane. The combined organic extracts were washed with brine and concentrated *in vacuo.* The residue was submitted to column chromatography (eluent: CH₂Cl₂/MeOH = 9/1), the free base was then converted to the hydrochloride salt (G1), to give a light yellow powder (34 mg). **NMR (¹H, CDCl₃):** δ 9.66 (bs, 1H), 7.74 (d, 1H), 7.11 (d, 1H), 7.68 (d, 1H) 7.23 (dd, 1H), 7.06 (d, 1H), 4.94 (s, 2H), 4.32 (t, 2H), 3.77 (s, 3H), 3.66 (m, 2H), 3.44 (m, 2H), 1.7-1.45 (m, 4H), 1.85 (d, 2H), 1.38 (d, 6H). **MS (*m*/*z*):** 463 [MH]⁺ (2Cl).

### Example 11: 7-Chloro-2-{4-methoxy-3-[2-(4-methyl-piperidin-1-yl)-ethoxy]phenyl}-2,3-dihydroisoindol-1-one hydrochloride

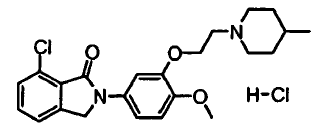

2-Chloro-methylbenzoic acid (200 mg) was treated as described in procedure G3 to give the corresponding ester (204 mg).
Methyl 2-chloro-6-methyl benzoate (204 mg) was treated as described in procedure G4 to give a colourless oil (180 mg) which consisted of a mixture of starting material and chloro derivative as judged by NMR. After heating this mixture and 4-methoxy-3-[2-(4-methyl-piperidin-1-yl)-ethoxy]-phenylamine hydrochloride (P5, 0.44 mmol) in dry DMF (1.5 ml) and dry 1,4-dioxane (0.7 ml) to 95 °C for 1h, 2,6-lutidine (0.37 mmol) was added. The mixture was heated to 95 °C for 2h, concentrated *in vacuo* and the residue submitted to column chromatography (eluent: CH₂Cl₂/MeOH = 95/5), Half of the free base was converted to the hydrochloride salt (G1), obtaining a faint yellow powder (27 mg): **NMR (¹H, d6-DMSO):** δ 9.9/9.7 (b, 1H), 7.75 (d, 1H), 7.63 (m, 2H), 7.53 (d, 1H), 7.30 (dd, 1H), 7.09 (d, 1H), 4.86 (s, 2H), 4.37 (t, 2H), 3.79 (s, 3H), 3.61 (m, 2H), 3.48 (m, 2H), 3.05 (m, 2H), 1.80 (m, 2H), 1.60 (m, 1H), 1.45 (m, 2H), 0.92 (d, 3H) (only data for major protonation isomer given). **MS (*m*/*z*):** 415 [MH]⁺ (1CI).

Examples 12 and 13 were prepared in analogy to Example 11:

| | | |
|---|---|---|
| **Example 12: 6-Chloro-2-{4-methoxy-3-[2-(4-methyl-piperidin-1-yl)-ethoxy]phenyl}-2,3-dihydroisoindol-1-one hydrochloride** | 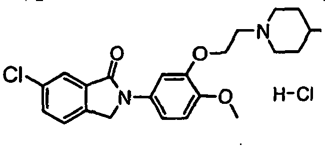 | **NMR (¹H, d6-DMSO):** δ 9.43 (bs, 1H), 7.72 (m, 4H), 7.36 (dd, 1H), 7.12 (d, 1H), 4.99 (s, 2H), 4.37 (t, 2H), 3.81 (s, 3H), 3.62/3.05 (d/t, 2/2H), 3.51 (t, 2H), 1.84/1.40 (d/t, 2/2H), 1.60 (m, 1H), 0.93 (d, 3H) (only data for major protonation isomer given). **MS (*m*/*z*):** 415 [MH]⁺ (1CI). |
| NOTE: Compound (E12) was obtained according to Example 11 using procedure G2 instead of G4 | | |

| | | |
|---|---|---|
| **Example 13: 5-Chloro-2-{4-methoxy-3-[2-(4-methyl-piperidin-1-yl)-ethoxy]phenyl}-2,3-dihydroisoindol-1-one hydrochloride** | 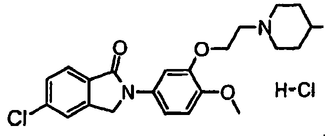 | **NMR (¹H, d6-DMSO):** δ 9.82 (bs, 1H), 7.78 (d, 1H), 7.76 (d, 1H), 7.71 (d, 1H), 7.60 (dd, 1H), 7.33 (dd, 1H), 7.09 (d, 1H), 4.99 (s, 2H), 4.37 (t, 2H), 3.79 (s, 3H), 3.60 (m, 2H), 3.40 (m, 2H), 3.04 (m, 2H), 1.82 (m, 2H), 1.60 (m, 1H), 1.44 (m, 2H), 0.91 (d, 3H) (only data for major protonation isomer given). **MS (*m*/*z*):** 415 [MH]⁺ (1Cl). |

### Example 14: 7-Methyl-2-{4-methoxy-3-[2-(4-methyl-piperidin-1-yl)-ethoxy]phenyl}-2,3-dihydroisoindol-1-one hydrochloride

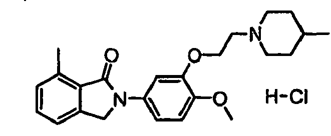

To a solution of 4-methoxy-3-[2-(4-methyl-piperidin-1-yl)-ethoxy]-phenylamine (P5, 0.74 mmol) in dry toluene (2 ml) at 0 °C was added AIMe₃ (2M in hexane, 2.0 mmol). After stirring the mixture at r.t. for 30 minutes, a solution of 7-methylphthalide (100 mg, 0.68 mmol) in CH₂Cl₂ (0.5 ml) was added at 0 °C. The solution was stirring at r.t. for 12 h. After removing the solvent under reduced pressure, the residue was diluted with CH₂Cl₂ and washed with NaOH (1 M) and brine. The organic phase was dried over Na₂SO₄, filtered and concentrated to dryness *in vacuo.* The crude was submitted to column chromatography to give a yellow oil (225 mg). To a solution of this material (168 mg, 0.4 mmol) in THF (1.5 ml) at 0 °C were added PPh₃ (0.8 mmol) and diisoropyl azodicarboxylate (0.8 mmol). The solution was stirred at 25 °C for 8 h, the solvent removed under reduced pressure and the residue purified by flash chromatography (CH₂Cl₂/MeOH = 9/1) followed by conversion to hydrochloride salt (G1) to give the title compound (89 mg): **NMR (¹H, (CD₃)₂CO)**: δ 7.87 (d, 1H), 7.48 (m, 2H), 7.42 (dd, 1H), 7.26 (dd, 1H), 7.05 (d, 1H), 4.92 (s, 2H), 4.69 (t, 2H), 3.86 (s, 3H), 3.65/3.1 (m/m, 2/2H), 3.47 (t, 2H), 2.70 (s, 3H), 1.9/1.8 (m/m, 2/2H), 1.70 (m, 1H), 0.99 (d, 3H) (only data for major protonation isomer given, hydrochloride proton not observed). **MS (*m*/*z*):** 395 [MH]⁺.

### Example 15: 6,7-Difluoro-2-{4-methoxy-3-[2-(4-methyl-piperidin-1-yl)-ethoxy]phenyl}-2,3-dihydroisoindol-1-one hydrochloride

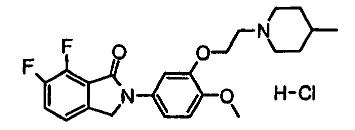

The title compound (36 mg) was obtained from 6,7-difluorophthalide (P11) (100 mg, 0.59 mmol) in analogy to Example 14: **NMR (¹H, d6-DMSO):** δ 9.35 (bs, 1H), 7.79 (m, 2H), 7.66 (d, 1H), 7.31 (dd, 1H), 7.08 (d, 1H), 4.94 (s, 2H), 4.31 (t, 2H), 3.76 (s, 3H), 3.58/3.27 (m/m, 2/2H), 3.47 (m, 2H), 1.80/1.37 (m/m, 2/2H), 1.57 (m, 1H), 0.89 (d, 3H) (only data for major protonation isomer given). **MS (*m*/*z*)**: 417 [MH]⁺.

### Example 16: 5,6-Dichloro-2-{4-methoxy-3-[2-(4-methyl-piperidin-1-yl)-ethoxy]phenyl}-2,3-dihydroisoindol-1-one hydrochloride

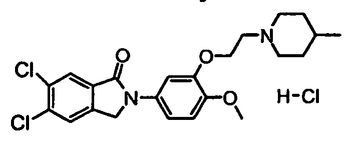

To a solution of 4-methoxy-3-[2-(4-methyl-piperidin-1-yl)-ethoxy]-phenylamine (P5, 4.2 mmol) in dry CH₂Cl₂ (20 ml) at 0 °C was added AIMe₃ (2M in hexane, 6.2 mmol). After 15 minutes 5,6-dichlorophthalide (P13, 0.83 g) was added and the solution allowed to warm to 25 °C. After 1.5 h additional 5,6-dichlorophthalide (P13, 0.07 g) was added. After additional 30 min NaOH (1 M aqueous) and CH₂Cl₂ were added. The resulting slurry was filtered through Celite, washing with some CH₂Cl₂. The CH₂Cl₂ layer (*ca*. 100 ml) was found to contain only a small fraction of the amide intermediate. The filtration residues and the aqueous phase were extracted using 1:4 isopropanol:EtOAc, filtered, the organic layer washed (brine), dried (Na₂SO₄), filtered and concentrated to dryness *in vacuo* to provide the amide intermediate (1.6 g) as an off-white solid. To this material was added PPh₃ (1.3 g) and THF (dry, 13 ml) followed by slow addition over 10 min at 50 °C of diisoropyl azodicarboxylate (1.0 ml). After 20 min water (3 drops) was added, afer additional 5 min volatiles evaporated and the residue submitted to column chromatography followed by salt formation (G1) to give the title compound as a colourless film (0.37 g material of high purity besides 0.93 g material of lesser purity).: **NMR (¹H, d6-DMSO):** δ 9.5 (bs, 1H), 8.00 (s, 1H), 7.95 (s, 1H), 7.70 (d, 1H), 7.33 (dd, 1H), 7.10 (d, 1H), 4.90 (s, 2H), 4.35 (t, 2H), 3.79 (s, 3H), 3.61 (m, 2H), 3.50 (m, 2H), 3.04 (m, 2H), 1.80 (m, 2H), 1.60 (m, 1H), 1.40 (m, 2H), 0.92 (d, 3H) (only data for major protonation isomer given). **MS (*m*/*z*):** 449 [MH]⁺ (2Cl).

### Example 17: 7-Fluoro-2-{4-methoxy-3-[2-(piperidin-1-yl)-ethoxy]phenyl}-2,3-dihydroisoindol-1-one hydrochloride

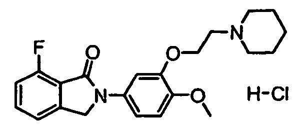

A solution of 3-fluorophthalic anhydride (166 mg, 1 mmol) and 4-methoxy-3-[2-(piperidin-1-yl)-ethoxy]-phenylamine (P2, 1 mmol) in dry DMF (1 ml) were heated in a microwave reactor to 200 W, 150 PSI for 10 min. After removing the solvent under reduced pressure, the residue was diluted with Et₂O/CH₂Cl₂ (4/1) and washed with water. The organic phase was dried over Na₂SO₄, filtered and concentrated to dryness *in vacuo.* The crude was submitted to column chromatography (CH₂Cl₂:MeOH=9:1) to give the corresponding phthalimide derivative (300 mg); **MS *(m*/*****z)**:* 399 [MH]⁺.
To a solution of this material (185 mg, 0.46 mmol) in dry THF (4 ml) at 0°C was added NaBH₄ (1.2 mmol). The solution was cooled at room temperature and stirred for 8 h. After removing the solvent under reduced pressure, the residue was diluted with CH₂Cl₂ and washed with sat. aqueous NaHCO₃ and brine. The organic phase was dried over Na₂SO₄, filtered and concentrated to dryness *in vacuo.* The crude was submitted to column chromatography (CH₂Cl₂:MeOH=9:1) to give a yellow oil (40 mg, 0.1 mmol)); **MS (*m*/*z*)**: 401 [MH]⁺. To this material was added CH₂Cl₂ (2 ml), Et₃SiH (0.8 mmol) and TFA (4 mmol). After 12 hours at room temperature volatiles were evaporated and the residue diluted with CH₂Cl₂ and washed with sat. aqueous NaHCO₃ and brine. The organic phase was dried over Na₂SO₄, filtered and concentrated to dryness *in vacuo.* The crude was submitted to column chromatography (CH₂Cl₂:MeOH=9:1) to give a mixture of compounds (22 mg) as judged by NMR.
The title compound (1.5 mg) was obtained from this mixture of isomers by HPLC purification: **NMR (¹H, (CD₃)₂CO):** δ 13.1 (bs, 1H), 7.80 (d, 1H), 7.67 (m, 1H), 7.50 (m, 2H), 7.23 (t, 1H), 7.07 (d, 1H), 5.05 (s, 2H), 4.69 (bs, 2H), 3.87 (s, 3H), 3.64 (bs, 2H), 3.47 (bs, 2H), 3.09 (bs, 2H), 2.18 (m, 2H), 1.85 (m, 2H), 1.50 (m, 2H).

### Example 18: 4-Fluoro-2-{4-methoxy-3-[2-(piperidin-1-yl)-ethoxy]phenyl}-2,3-dihydroisoindol-1-one hydrochloride

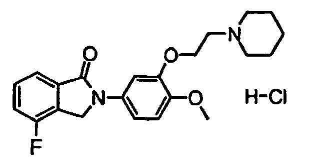

The title compound (4 mg) was obtained by HPLC purification from the mixture of compounds described in the preparation of Example 17: **NMR (¹H, (CD₃)₂CO):** δ 7.86 (d, 1H), 7.7/7.6 (m, 2H), 7.55 (m, 2H), 7.43 (t, 1H), 7.07 (d, 1H), 5.10 (s, 2H), 4.70 (t, 2H), 3.88 (s, 3H), 3.66 (d, 2H), 3.49 (t, 2H), 3.12 (t, 2H), 2.20 (m, 2H), 1.85 (m, 2H), 1.35 (m, 2H), (hydrochloride proton not observed).

### Example 19: 5,7-Dimethyl-2-{4-methoxy-3-[2-(4-methyl-piperidin-1-yl)-ethoxy]phenyl}-2,3-dihydroisoindol-1-one hydrochloride

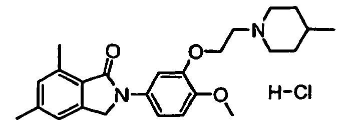

Methyl 2,4,6-trimethyl benzoate (12 mmol) was treated as described in procedure G2 to give a colourless oil (2.6 g) which consisted of a mixture of compounds as judged by NMR. A portion of this material (0.22 g) and 4-methoxy-3-[2-(4-methyl-piperidin-1-yl)-ethoxy]-phenylamine hydrochloride (P5, 1.3 mmol) in dry DMF (2.5 ml) were heated in a microwave reactor to 150 °C for 15 min. The mixture was concentrated *in vacuo,* and the residue submitted to column chromatography, follwed by conversion to the hydrochloride salt (G1). This was extracted with hot EtOAc. On standing at 25 °C the title compound crystallised from this solution as a faint yellow powder (15 mg): **NMR (¹H, d4-MeOH):** δ 7.79 (d, 1H), 7.22-7.26 (m, 2H), 7.10-7.15 (m, 2H), ca. 4.8 (under H₂O resonance, 2H), 4.41 (t, 2H), 3.91 (s, 3H), 3.76 (bd, 2H), 3.58 (t, 2H), 3.13 (bt, 2H), 2.66 (s, 3H), 2.43 (s, 3H), 1.90-2.04 (m, 2H), 1.70-1.82 (m, 1H), 1.45-1.57 (m, 2H), 1.05 (d, 5H)(only data for major protonation isomer given). **MS (*m*/*z*): 409 [MH]⁺**.

Examples 20-23 were prepared in analogy to Example 11 but using using procedure G2 instead of G4:

| | | |
|---|---|---|
| **Example 20 (E20): 6,7-Dichloro-2-{4-methoxy-3-[2-(4-methyl-piperidin-1-yl)-ethoxy]phenyl}-2,3-dihydroisoindol-1-one hydrochloride** | 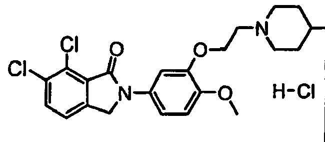 | **NMR (¹H, d6-DMSO): δ** 7.78 (m, 2H), 7.54 (d, 1H), 7.22 (dd, 1H), 7.10 (d, 1H), 4.87 (s, 2H), 4.39 (t, 2H), 3.88 (s, 3H), 3.74 (d, 2H), 3.56 (t, 2H), 3.11 (t, 2H), 1.97 (d, 2H), 1.49 (t, 2H), 1.75 (m, 1H), 1.02 (d, 3H) (only data for major protonation isomer given, hydrochloride proton not observed). **MS *(m*/*z):*** 449 [MH]⁺ (2Cl). |
| **Example 21 (E21): 5-Fluoro-2-{4-methoxy-3-[2-(4-methyl-piperidin-1-yl)-ethoxy]phenyl}-** | 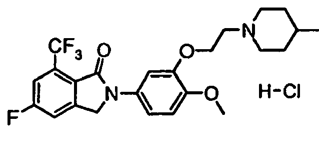 | **NMR (¹H, d6-DMSO):** δ 7.84 (d, 1H), 7.70 (m, 2H), 7.26 (dd, 1H), 7.15 (d, 1H), 5.04 (s, 2H), 4.44 (t, 2H), 3.94 (s, 3H), 3.78 (bd, 2H), |
| **7-trifluoromethyl-2,3-dihydroisoindol-1-one hydrochloride** | | 3.61 (t, 2H), 3.14 (bt, 2H), 2.01 (bd, 2H), 1.77 (m, 1H), 1.52 (dq, 2H), 1.07 (d, 3H) (only data for major protonation isomer given, hydrochloride proton not observed). **MS *(m*/*z):*** 467 [MH]⁺. |
| **Example 22 (E22): 7-Chloro-4,5-difluoro-2-{4-methoxy-3-[2-(4-methyl-piperidin-1-yl)-ethoxy]phenyl}-2,3-dihydroisoindol-1-one hydrochloride** | 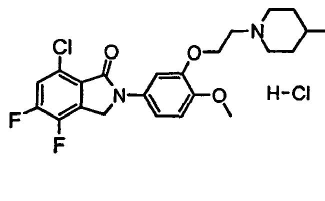 | **NMR (¹H, d6-DMSO):** δ 7.85 (dd, 1H), 7.54 (d, 1H), 7.31 (dd, 1H), 7.01 (d, 1H), 5.09 (s, 2H), 4.07 (t, 2H), 3.76 (s, 3H), 2.89 (m, 2H), 2.68 (t, 2H), 2.01 (m, 2H), 1.56 (m, 2H), 1.31 (m, 1H), 1.12 (m, 2H), 0.87 (d, 3H) (only data for major protonation isomer given, hydrochloride proton not observed). **MS *(m*/*z):*** 451 [MH]⁺ (1Cl). |
| **Example 23 (E23):4-Fluoro-2-{4-methoxy-3-[2-(4-methyl-piperidin-1-yl)-ethoxy]phenyl}-7-trifluoromethyl-2,3-dihydroisoindol-1-one hydrochloride** | 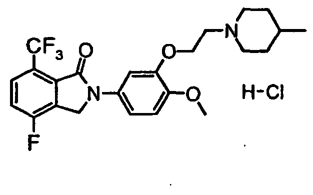 | **NMR (¹H, d6-DMSO):** δ 9.55 (bs, 1H), 7.97 (dd, 1H), 7.76 (d, 1H), 7.71 (t, 1H), 7.38 (dd, 1H), 7.10 (d, 1H), 5.14 (s, 2H), 4.37 (t, 2H), 3.81 (s, 3H), 3.59 (m, 2H), 3.50 (m, 2H), 3.05 (q, 2H), 1.82 (bd, 2H), 1.60 (bm, 1H), 1.42 (q, 2H), 0.92 (d, 3H) (only data for major protonation isomer given). **MS** ***(mlz)**:* 467 [MH]⁺. |

### Example 24: 7-Cyano-2-{4-methoxy-3-[2-(4-methyl-piperidin-1-yl)-ethoxy]phenyl}-2,3-dihydroisoindol-1-one hydrochloride

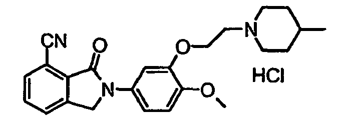

7-Bromo-2-{4-methoxy-3-[2-(4-methyl-piperidin-1-yl)-ethoxy]phenyl}-2,3-dihydroisoindol-1-one (free base, 0.15 g, prepared as described for Example 3) was heated at reflux in the presence of CuCN (61 mg) in dry DMF (7 ml) for 2 h. The mixture was allowed to cool, diluted with half conc. aqueous Na₂CO₃ and extracted with DCM (2 portions), the aqueous layer further diluted with brine and extracted again (EtOAc). The combined organic extracts were concentrated *in vacuo,* the residue purified by column chromatography followed by preparative HPLC, follwed by conversion to the hydrochloride salt (G1) to give the title compound (14 mg) as a yellow solid: **NMR (¹H, d4-MeOH):** δ 7.94 (2d, 2H), 7.90 (t, 1H), 7.83 (t, 1H), 7.28 (dd, 1H), 7.14 (d, 1H), 5.02 (s, 2H), 4.43 (t, 2H), 3.92 (s, 3H), 3.77 (d, 2H), 3.60 (t, 2H), 3.12 (bt, 2H), 1.93-2.02 (m, 2H), 1.66-1.82 (m, 1H), 1.43-1.58 (m, 2H), 1.04 (d, 3H)(only data for major protonation isomer given). **MS (*m*/*z*)*:*** 406 [MH]⁺.

### Example 25: 5,7-Dichloro-2-{4-methoxy-3-[2-(4-methyl-piperidin-1-yl)-ethoxy]phenyl}-6-methyl-2,3-dihydroisoindol-1-one hydrochloride

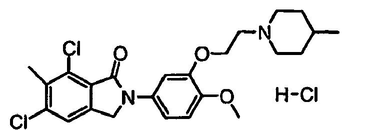

To a solution of 4-methoxy-3-[2-(4-methyl-piperidin-1-yl)-ethoxy]-phenylamine (P5, 0.18 g) in dry CH₂Cl₂ (2 ml) at 0 °C was added AIMe₃ (2M in hexane, 0.54 ml). After 15 minutes 5,7-dichloro-6-methyl-phthalide (P17, 0.18 g) was added and the solution allowed to warm to 25°C. After 5 h silica gel, water (few drops), 10% conc. aqueous NH₃/MeOH:CH₂Cl₂ 1:10 were added with vigorous stirring, the resulting slurry removed by filtration and washed well with 10% conc. aqueous NH₃/MeOH:CH₂Cl₂ 1:10. The solution thus obtained was concentrated *in vacuo* and the residue submitted to column chromatography to provide the amide intermediate (0.26 g) as a red gum. To this material was added PPh₃ (0.19 g) and THF (dry, 2 ml) followed by slow addition over 3 min at 50 °C of diisoropyl azodicarboxylate (0.14 ml). After 10 min the mixture was allowed to cool to 25 °C, volatiles evaporated and the residue submitted to repeated column chromatography followed by salt formation (G1) to give the title compound as a colourless solid (0.15 g):
**NMR (¹H, d4-MeOH):** δ 7.76 (d, 1H), 7.65 (s, 1H), 7.23 (dd, 1H), 7.10 (d, 1H), 4.86 (s, 2H), 4.40 (t, 2H), 3.90 (s, 3H), 3.75 (d, 2H), 3.57 (t, 2H), 3.10 (t, 2H), 2.56 (s, 3H), 1.97 (d, 2H), 1.60-1.80 (m, 1H), 1.42-1.56 (m, 2H), 1.03 (d, 3H) (only data for major protonation isomer given). **MS (*m*/*z*)*:*** 463 [MH]⁺ (2Cl).

### Example 26: 5,6-Dichloro-2-{4-methoxy-3-[2-(4-methyl-piperidin-1-yl)-ethoxy]phenyl}-7-methyl-2,3-dihydroisoindol-1-one hydrochloride

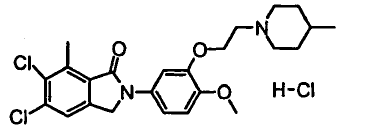

The title compound (yellow solid, 0.14 g) was prepared in an analogous manner to the compound of Example 25, starting from 5,6-dichloro-7-methyl-phthalide (P19, 0.13 g):
**NMR (¹H, d4-MeOH):** δ 7.8 (d, 1H), 7.7 (s, 1H), 7.3 (dd, 1H), 7.17 (d, 1H), 4.9 (s, 2H), 4.45 (t, 2H), 3.94 (s, 3H), 3.8 (d, 2H), 3.6 (t, 2H), 3.15 (t, 2H), 2.6 (s, 3H), 2.0 (d, 2H), 1.80 (m, 1H), 1.45-1.62 (m, 2H), 1.09 (d, 3H) (only data for major protonation isomer given).
**MS (*m*/*z*):** 463 [MH]⁺ (2Cl).

### Example 27: 5,7-Dichloro-2-{4-methoxy-3-[2-(4,4-dimethyl-piperidin-1-yl)-ethoxy]phenyl}-2,3-dihydroisoindol-1-one hydrochloride

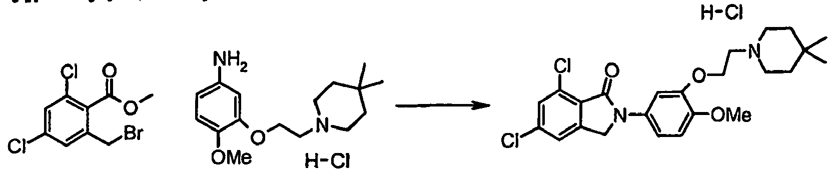

2-(Bromomethyl)-4,6-dichlorobenzoic acid methyl ester (200 mg, 50% purity, 0.33 mmol, cf. intermediate in Preparation 14), and 3-[2-(4,4-dimethylpiperidin-1-yl)ethoxy]-4-methoxyphenylamine hydrochloride (124 mg, 0.39 mmol) were dissolved in dry DMF (4 ml) and allowed to react in a microwawe oven at 95 °C for 20 min. A saturated solution of NaHCO₃ was added and the mixture was extracted with dichloromethane. The organic phase was washed with brine, dried over Na₂SO₄ and concentrated *in vacuo.* The residue was purified by flash chromatography (CH₂Cl₂/MeOH 9/1), to give 34 mg of the title compound, which was tranformed into the hydrochloride salt (40 mg, white solid) by addition of HCl in Et₂O. **NMR (¹H, DMSO-d6):** δ 9.68 (bs, 1H), 7.74 (s, 1H), 7.71 (s, 2H), 7.27 (dd, 1H), 7.09 (d, 1H), 4.94 (s, 2H), 4.36 (t, 2H), 3.78 (s, 3H), 3.53 (t, 2H), 3.46 (d, 2H), 3.18 (q, 2H), 1.69 (t, 2H), 1.53 (d, 2H), 1.08 (s, 3H), 0.95 (s, 3H). **MS (*m*/*z*):** 463 [MH]⁺ (2Cl).

### Example 28: 5,7-Dichloro-2-{4-methoxy-3-[2-(azepan-1-yl)-ethoxy]phenyl}-2,3-dihydroisoindol-1-one hydrochloride

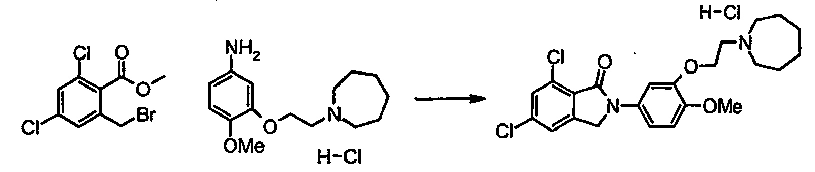

2-(Bromomethyl)-4,6-dichlorobenzoic acid methyl ester (232 mg, 60% purity, 0.46 mmol, cf. intermediate in Preparation 14) and 3-(2-azepan-1-ylethoxy)-4-methoxyphenylamine hydrochloride (140 mg, 0.46 mmol) were dissolved in dry DMF (4 ml) and reacted in a microwawe oven at 130 °C for 20 min. A saturated solution of NaHCO₃ was added and the mixture was extracted with dichloromethane. The organic phase was washed with brine, dried over Na₂SO₄ and concentrated *in vacuo.* The residue was purified by flash chromatography (CH₂Cl₂/MeOH 9/1), to give 77 mg of the title compound, which was converted into the hydrochloride salt (83 mg, white solid) by addition of HCl in Et₂O. **NMR (¹H, CDCl₃):** δ 9.70 (bs, 1H), 7.72 (d, 1H), 7.70 (d, 1H), 7.68 (d, 1H), 7.23 (dd, 1H), 7.06 (d, 1H), 4.92 (s, 2H), 4.32 (t, 2H), 3.77 (s, 3H), 3.53 (q, 2H), 3.45 (m, 2H), 3.26 (m, 2H), 1.80 (m, 4H), 1.60 (m, 4H). **MS (*m*/*z*)**: 449 [MH]⁺ (2CI).

### Example 29: 5,7-Dichloro-2-{4-methoxy-3-[2-(2-methyl-piperidin-1-yl)-ethoxy]phenyl}-2,3-dihydroisoindol-1-one hydrochloride

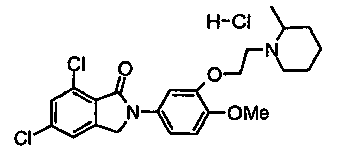

The title compound was prepared from 2-methyl-piperidine following the procedure used for Example 10. **NMR (¹H, DMSO-d6):** δ 9.8 (bs, 1H), 7.78 (m, 3H), 7.28 (dd, 1H), 7.13 (d, 1H), 4.99 (s, 2H), 4.39 (m, 2H), 3.82 (s, 3H), 3.7-3.2 (m, 5H), 2.0-1.40 (m, 6H), 1.40 (d, 3H). **MS (*m*/*z*):** 449 [MH]⁺(2Cl).

### Example 30: 6-{4-Methoxy-3-[2-(4-methyl-piperidin-1-yl)-ethoxy]-phenyl}-2-methyl-4-trifluoromethyl-6,7-dihydro-pyrrolo[3,4-b]pyridin-5-one hydrochloride

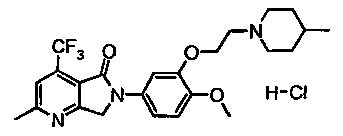

Ethyl 2,6-dimethyl-4-trifluoromethylpyridine-3-carboxylate (Katsumaya, I. *et al., Synthesis* **1997,** *11,* 1321-1324, 1.0 g) was treated as described in Procedure G2 to provide a colourless oil (0.55 g) containing ca. 50 mol% ethyl 2-bromomethyl-6-methyl-4-trifluoromethylpyridine-3-carboxylate. This material (0.33 g) and 4-methoxy-3-[2-(4-methyl-piperidin-1-yl)-ethoxy]-phenylamine hydrochloride (P5, 1.25 mmol) in dry DMF (2.5 ml) were heated in a microwave reactor to 150 °C for 15 min. The mixture was concentrated *in vacuo,* and the residue submitted to column chromatography, follwed by trituration with MeOH:H₂O 5:1 and subsequently a small volume of MeOH (ca. 3 ml), followed by conversion to the hydrochloride salt (G1) to give the title compound as an orange solid (0.15 g): **NMR (¹H, d4-MeOH)**: δ 7.84 (d, 1H), 7.77 (s, 1H), 7.32 (dd, 1H), 7.18 (d, 1H), 5.04 (s, 2H), 4.47 (t, 2H), 3.95 (s, 3H), 3.81 (d, 2H), 3.63 (t, 2H), 3.14 (t, 2H), 2.80 (s, 3H), 1.99 (d, 2H), 1.70-1.90 (m, 1H), 1.45-1.60 (m, 2H), 1.09 (d, 3H) (only data for major protonation isomer given). **MS *(m*/*z)*:** 464 [MH]⁺.

### Example 31: 5,7-Dichloro-4-fluoro-2-{4-methoxy-3-[2-(4-methyl-piperidin-1-yl)-ethoxy]phenyl}-2,3-dihydroisoindol-1-one hydrochloride

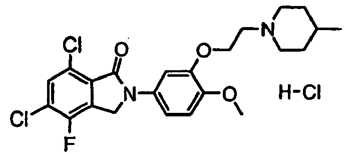

The title compound was prepared in analogy to Example 11 but using using procedure G2 instead of G4: **NMR (¹H, d6-DMSO):** δ 7.77 (m, 2H), 7.32 (dd, 1H), 7.18 (d, 1H), 5.05 (s, 2H), 4.45 (t, 2H), 3.94 (s, 3H), 3.81/3.18 (d/t, 4H), 3.60 (t, 2H), 2.05/1.59 (d/t, 4H), 1.78 (m, 1H), 1.09 (d, 3H) (only data for major protonation isomer given). **MS (*m*/*z*)**: 467 [MH]⁺ (2Cl).

## Claims

1. A compound of formula (I) or a pharmaceutically acceptable salt thereof: 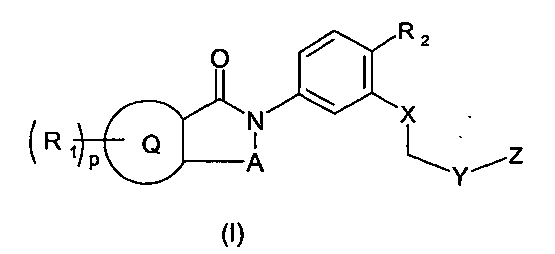 wherein:
R₁ is halogen, cyano, C₁₋₆alkyl, C₃₋₇cycloalkyl, C₃₋₇cycloalkyloxy, C₁₋₆alkoxy, C₁₋₆alkylthio, hydroxy, amino, mono- or di-C₁₋₆alkylamino, an N-linked 4 to 7 membered heterocyclic group, nitro, haloC₁₋₆alkyl, haloC₁₋₆alkoxy, aryl, -COOR₃, -COR₄ (wherein R₃ and R₄, are independently hydrogen or C₁₋₆alkyl) or -COR₅ (wherein R₅ is amino, mono- or di-C₁₋₆alkylamino or an N-linked 4 to 7 membered heterocyclic group);
p is 0, 1 or 2 or 3;
Q is a 6- membered aromatic group or a 6-membered heteroaromatic group;
A is -(CH₂-CH₂)-, -(CH=CH)-, or a group -(CHR₇)- wherein R₇ is hydrogen, halogen, hydroxy, cyano, nitro, C₁₋₆alkyl, C₃₋₇cycloalkyl, C₃₋₇cycloalkyloxy, haloC₁₋₆alkyl, C₁₋₆ alkoxy, haloC₁₋₆alkoxy or C₁₋₆alkylthio;
R₂ is hydrogen, halogen, hydroxy, cyano, nitro, C₁₋₆alkyl, C₁₋₆alkanoyl, C₃₋₇cycloalkyl, C₃₋₇cycloalkyloxy, haloC₁₋₆alkyl, C₁₋₆alkoxy, haloC₁₋₆alkoxy, C₁₋₆alkylthio, amino, mono- or di-C₁₋₆alkylamino or an N-linked 4 to 7 membered heterocyclic group;
X is oxygen, sulfur, -CH₂- or NR₈ wherein R₈ is hydrogen or C₁₋₆alkyl;
Y is a single bond, -CH₂-, -(CH₂)₂- or-CH=CH-; and
Z is an optionally substituted N-linked heterocyclic group or a C-linked 4 to 7 membered heterocyclic group containing at least one nitrogen, or Z is -NR₉R₁₀ where Rg and R₁₀ are independently hydrogen or C₁₋₆alkyl.

2. A compound as claimed in claim 1, wherein when R₇ is hydrogen.

3. A compound as claimed in claim 1 or claim 2, wherein A is CH₂-.

4. A compound as claimed in any of claims 1-3, wherein Q is phenyl.

5. A compound as claimed in any of claims 1-4, wherein p is 1, 2 or 3, and R₁ is/are halogen (particularly chloro or fluoro), C₁₋₆alkyl (particularly methyl) or CF₃.

6. A compound as claimed in any of claims 1-5, wherein when R₁ is attached at the position marked below with an asterisk, R₁ is fluoro:

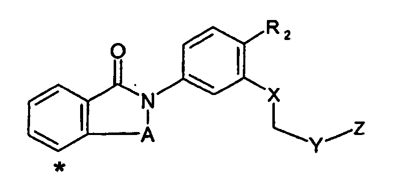

7. A compound as claimed in any of claims 1-6, wherein when Q is phenyl and p is 1, R₁ is attached at the position marked below with an asterisk:

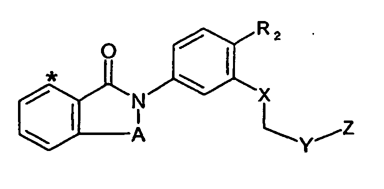

8. A compound as claimed in any of claims 1-7, wherein when Q is phenyl and p is 2 or 3, R₁ is attached at two or more of the positions marked below with arrows:

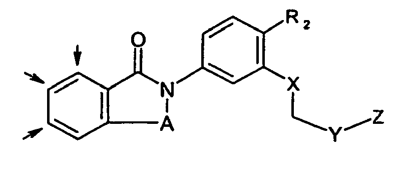

9. A compound as claimed in any of claims 1-8, wherein R₂ is C₁₋₆alkoxy, particularly methoxy.

10. A compound as claimed in any of claims 1-9, wherein X is oxygen.

11. A compound as claimed in any of claims 1-10, wherein Y is -CH₂-.

12. A compound as claimed in any of claims 1-11, wherein Z is an optionally substituted N-linked 4 to 7 membered heterocycle, in particular optionally substituted piperidyl.

13. A compound as claimed in claim 1 having the formula (Ia): 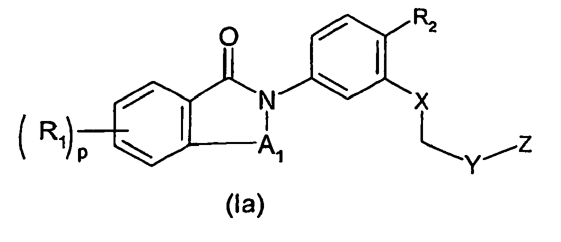 wherein R₁, p, R₂, X, Y, Z, are as defined in any of claims 1-12 and A₁ is -CH₂- or - HC(Me)-.

14. A compound as claimed in claim 1, which is
2-[4-Methoxy-3-(2-piperidin-1-yl-ethoxy)phenyl]-2,3-dihydroisoindol-1-one
6-Fluoro-2-[4-methoxy-3-(2-piperidin-1-yl-ethoxy)phenyl]-2,3-dihydroisoindol-1-one
7-Bromo-2-{4-methoxy-3-[2-(4-methyl-piperidin-1-yl)-ethoxy]phenyl}-2,3-dihydroisoindol-1-one hydrochloride
7-Chloro-2-{4-methoxy-3-[2-(4-methyl-piperidin-1-yl)-ethoxy]phenyl}-3-methyl-2,3-dihydroisoindol-1-one
2-{4-Methoxy-3-[2-(4-methyl-piperidin-1-yl)-ethoxy]phenyl}-7-trifluoromethyl-2,3-dihydroisoindol-1-one
5,7-Dichloro-2-{4-methoxy-3-[2-(4-methyl-piperidin-1-yl)-ethoxy]phenyl}-2,3-dihydroisoindol-1-one
7-Chloro-2-[4-methoxy-3-(2-piperidin-1-yl-ethoxy)phenyl]-2,3-dihydroisoindol-1-one
6-Chloro-2-[4-methoxy-3-(2-piperidin-1-yl-ethoxy)phenyl]-2,3-dihydroisoindol-1-one hydrochloride
5-Chloro-2-[4-methoxy-3-(2-piperidin-1-yl-ethoxy)phenyl]-2,3-dihydroisoindol-1-one
5,7-Dichloro-2-{4-methoxy-3-[2-(*cis*-2,6-dimethyl-piperidin-1-yl)-ethoxy]phenyl}-2,3-dihydroisoindol-1-one
7-Chloro-2-{4-methoxy-3-[2-(4-methyl-piperidin-1-yl)-ethoxy]phenyl}-2,3-dihydroisoindol-1-one
6-Chloro-2-{4-methoxy-3-[2-(4-methyl-piperidin-1-yl)-ethoxy]phenyl}-2,3-dihydroisoindol-1-one
5-Chloro-2-{4-methoxy-3-[2-(4-methyl-piperidin-1-yl)-ethoxy]phenyl}-2,3-dihydroisoindol-1-one
7-Methyl-2-{4-methoxy-3-[2-(4-methyl-piperidin-1-yl)-ethoxy]phenyl}-2,3-dihydroisoindol-1-one
6,7-Difluoro-2-{4-methoxy-3-[2-(4-methyl-piperidin-1-yl)-ethoxy]phenyl}-2,3-dihydroisoindol-1-one
5,6-Dichloro-2-{4-methoxy-3-[2-(4-methyl-piperidin-1-yl)-ethoxy]phenyl}-2,3-dihydroisoindol-1-one
7-Fluoro-2-{4-methoxy-3-[2-(piperidin-1-yl)-ethoxy]phenyl}-2,3-dihydroisoindol-1-one
4-Fluoro-2-{4-methoxy-3-[2-(piperidin-1-yl)-ethoxy]phenyl}-2,3-dihydroisoindol-1-one
5,7-Dimethyl-2-{4-methoxy-3-[2-(4-methyl-piperidin-1-yl)-ethoxy]phenyl)-2,3-dihydroisoindol-1-one
6,7-Dichloro-2-(4-methoxy-3-[2-(4-methyl-piperidin-1-yl)-ethoxy]phenyl)-2,3-dihydroisoindol-1-one
5-Fluoro-2-{4-methoxy-3-[2-(4-methyl-piperidin-1-yl)-ethoxy]phenyl}-7-trifluoromethyl-2,3-dihydroisoindol-1-one
7-Chloro-4,5-difluoro-2-{4-methoxy-3-[2-(4-methyl-piperidin-1-yl)-ethoxy]phenyl}-2,3-dihydroisoindol-1-one
4-Fluoro-2-{4-methoxy-3-[2-(4-methyl-piperidin-1-yl)-ethoxy]phenyl}-7-trifluoromethyl-2,3-dihydroisoindol-1-one
5,7-Dichloro-2-(4-methoxy-3-[2-(4,4-dimethyl-piperidin-1-yl)-ethoxy]phenyl)-2,3-dihydroisoindol-1-one
5,7-Dichloro-2-{4-methoxy-3-[2-(azepan-1-yl)-ethoxy]phenyl}-2,3-dihydroisoindol-1-one
5,7-Dichloro-2-{4-methoxy-3-[2-(2-methyl-piperidin-yl)-ethoxy]phenyl}-2,3-dihydroisoindol-1-one
6-{4-Methoxy-3-[2-(4-methyl-piperidin-1-yl)-ethoxy]-phenyl}-2-methyl-4-trifluoromethyl-6,7-dihydro-pyrrolo[3,4-b]pyridin-5-one
5,7-Dichloro-4-fluoro-2-{4-methoxy-3-[2-(4-methyl-piperidin-1-yl)-ethoxy]phenyl}-2,3-dihydroisoindol-1-one
or a pharmaceutically acceptable salt thereof.

15. A process for the preparation of a compound as claimed in any of claims 1-14 or a pharmaceutical acceptable salt thereof, which process comprises:
(a) reacting a compound of formula (II): 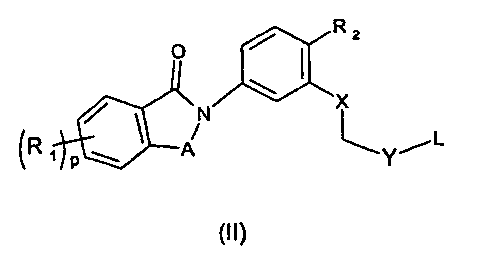 wherein R₁, R₂, p, A, X, and Y are as defined for formula (I), and L is a leaving group, with a compound of formula (III):
Z-H (III)
wherein Z is as defined for formula (I); or
(b) reacting a compound of formula (IV): 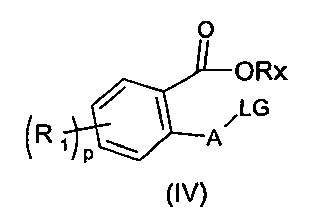 wherein Rx is alkyl and LG is a suitable leaving group, with a compound of formula (V) or a corresponding salt: 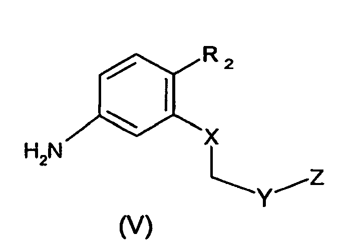 or
(c) reacting a compound of formula (VI): 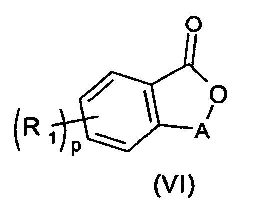
with a compound of formula (V) in the presence of AlMe₃ or a similar oxophilic reagent followed by treatment of the resulting amide under dehydrating conditions, e.g. with PPh₃ and dialkylazadicarboxylate;
and thereafter, for either process (a), process (b) or process (c), optionally followed by:
• removing any protecting groups; and/or
• converting a compound of formula (I) into another compound of formula (I); and/or
• forming a pharmaceutically acceptable salt.

16. A pharmaceutical composition comprising a compound as defined in any of claims 1-14 or a pharmaceutically acceptable salt thereof, and a pharmaceutically acceptable carrier or excipient.

17. A process for preparing a pharmaceutical composition as defined in claim 16, the process comprising mixing a compound as defined in any of claims 1-14 or a pharmaceutically acceptable salt thereof and a pharmaceutically acceptable carrier or excipient.

18. A compound as defined in any of claims 1-14 or a pharmaceutically acceptable salt thereof for use as a therapeutic substance.

19. A compound as defined in any of claims 1-14 for use in the treatment of a CNS disorder such as depression or anxiety.

20. Use of a compound as claimed in any of claims 1-14 or a pharmaceutically acceptable salt thereof in the manufacture of a medicament for use in the treatment of a CNS disorder.

21. The use as claimed in claim 22, wherein the CNS disorder is depression or anxiety.

## Patentansprüche

1. Verbindung der Formel (I) oder ein pharmazeutisch verträgliches Salz davon: 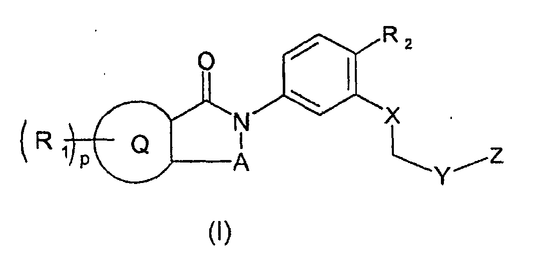 wobei:
R₁ Halogen, Cyano, C₁₋₆-Alkyl, C₃₋₇-Cycloalkyl, C₃₋₇-Cycloalkyloxy, C₁₋₆-Alkoxy, C₁₋₆-Alkylthio, Hydroxy, Amino, Mono- oder Di-C₁₋₆-alkylamino, ein N-gebundener 4 bis 7-gliedriger heterocyclischer Rest, Nitro, Halogen-C₁₋₆-alkyl, Halogen-C₁₋₆-alkoxy, Aryl, -COOR₃, -COR₄ (wobei R₃ und R₄ unabhängig Wasserstoff oder C₁₋₆-Alkyl sind) oder -COR₅ (wobei R₅ Amino, Mono- oder Di-C₁₋₆-alkylamino oder ein N-gebundener 4 bis 7-gliedriger heterocyclischer Rest ist) ist;
p gleich 0, 1 oder 2 oder 3 ist;
Q ein 6-gliedriger aromatischer Rest oder ein 6-gliedriger heteroaromatischer Rest ist;
A -(CH₂-CH₂)-, -(CH=CH)- oder ein Rest -(CHR₇)- ist, wobei R₇ Wasserstoff, Halogen, Hydroxy, Cyano, Nitro, C₁₋₆-Alkyl, C₃₋₇-Cylcoalkyl, C₃₋₇-Cycloalkyloxy, Halogen-C₁₋₆-alkyl, C₁₋₆ Alkoxy, Halogen-C₁₋₆-alkoxy oder C₁₋₆-Alkylthio ist;
R₂ Wasserstoff, Halogen, Hydroxy, Cyano, Nitro, C₁₋₆-Alkyl, C₁₋₆-Alkanoyl, C₃₋₇-Cycloalkyl, C₃₋₇-Cycloalkyloxy, Halogen-C₁₋₆-alkyl, C₁₋₆-Alkoxy, Halogen-C₁₋₆-alkoxy, C₁₋₆-Alkylthio, Amino, Mono- oder Di-C₁₋₆-alkylamino oder ein N-gebundener, 4 bis 7-gliedriger heterocyclischer Rest ist;
X Sauerstoff, Schwefel, -CH₂- oder NR₈ ist, wobei R₈ Wasserstoff oder C₁₋₆-Alkyl ist;
Y eine Einfachbindung, -CH₂-, -(CH₂)₂- oder -CH=CH- ist; und
Z ein gegebenenfalls substituierter, N-gebundener, heterocyclischer Rest oder ein C-gebundener, 4 bis 7-gliedriger heterocyclischer Rest, der mindestens einen Stickstoff enthält, ist, oder Z -NR₉R₁₀ ist, wobei R₉ und R₁₀ unabhängig Wasserstoff oder C₁₋₆-Alkyl sind.

2. Verbindung gemäß Anspruch 1, wobei R₇ Wasserstoff ist.

3. Verbindung gemäß Anspruch 1 oder Anspruch 2, wobei A CH₂- ist.

4. Verbindung gemäß einem der Ansprüche 1 bis 3, wobei Q Phenyl ist.

5. Verbindung gemäß einem der Ansprüche 1 bis 4, wobei p 1, 2 oder 3 ist und R₁ Halogen (insbesondere Chlor oder Fluor), C₁₋₆-Alkyl (insbesondere Methyl) oder CF₃ ist/sind.

6. Verbindung gemäß einem der Ansprüche 1 bis 5, wobei R₁ Fluor ist, wenn R₁ an der nachstehend mit einem Stern markierten Position gebunden ist:

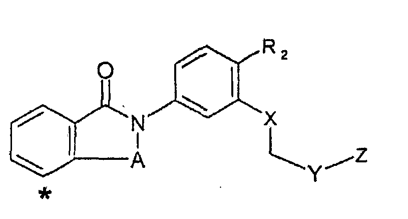

7. Verbindung gemäß einem der Ansprüche 1 bis 6, wobei R₁ an der nachstehend mit einem Stern markierten Position gebunden ist, wenn Q Phenyl ist und p 1 ist:

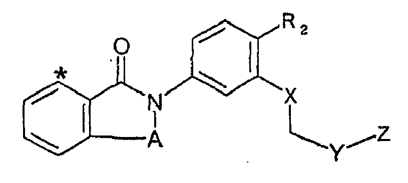

8. Verbindung gemäß einem der Ansprüche 1 bis 7, wobei R₁ an zwei oder mehreren nachstehend mit Pfeilen markierten Positionen gebunden ist, wenn Q Phenyl ist und p 2 oder 3 ist:

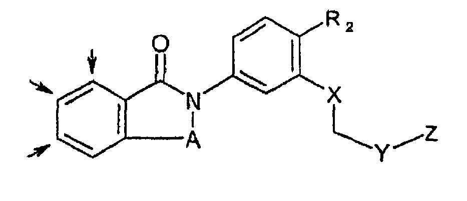

9. Verbindung gemäß einem der Ansprüche 1 bis 8, wobei R₂ C₁₋₆ Alkoxy, insbesondere Methoxy, ist.

10. Verbindung gemäß einem der Ansprüche 1 bis 9, wobei X Sauerstoff ist.

11. Verbindung gemäß einem der Ansprüche 1 bis 10, wobei Y -CH₂- ist.

12. Verbindung gemäß einem der Ansprüche 1 bis 11, wobei Z ein gegebenenfalls substituierter, N-gebundener, 4 bis 7-gliedriger Heterocyclus, insbesondere gegebenenfalls substituiertes Piperidyl, ist.

13. Verbindung gemäß Anspruch 1 mit der Formel (Ia): 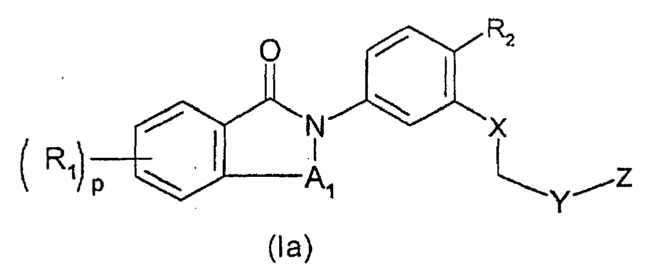 wobei R₁, p, R₂, X, Y, Z wie in einem der Ansprüche 1 bis 12 definiert sind und A₁ -CH₂- oder -HC(Me)- ist.

14. Verbindung gemäß Anspruch 1, welche
2-[4-Methoxy-3-(2-piperidin-1-yl-ethoxy)phenyl]-2,3-dihydroisoindol-1-on,
6-Fluor-2-[4-methoxy-3-(2-piperidin-1-yl-ethoxy)phenyl]-2,3-dihydroisoindol-1-on,
7-B rom-2- {4-methoxy-3-[2-(4-methyl-piperidin-1-yl)-ethoxy]phenyl}-2,3-dihydroisoindol-1-on-Hydrochlorid,
7-Chlor-2- {4-methoxy-3-[2-(4-methyl-piperidin-1-yl)-ethoxy]phenyl}-3-methyl-2,3-dihydroisoindol-1-on,
2- {4-Methoxy-3-[2-(4-methyl-piperidin-1-yl)-ethoxy]phenyl}-7-trifluormethyl-2,3-dihydroisoindol-1-on,
5,7-Dichlor-2- {4-methoxy-3-[2-(4-methyl-piperidin-1-yl)-ethoxy]phenyl}-2,3-dihydroisoindol-1-on,
7-Chlor-2-[4-methoxy-3-(2-piperidin-1-yl-ethoxy)phenyl]-2,3-dihydroisoindol-1-on,
6-Chlor-2-[4-methoxy-3-(2-piperidin-1-yl-ethoxy)phenyl]-2,3-dihydroisoindol-1-on-Hydrochlorid,
5-Chlor-2-[4-methoxy-3-(2-piperidin-1-yl-ethoxy)phenyl]-2,3-dihydroisoindol-1-on,
5,7-Dichlor-2- {4-methoxy-3-[2-(cis-2,6-dimethyl-piperidin-1-yl)-ethoxy]phenyl}-2,3-dihydroisoindol-1-on,
7-Chlor-2-{4-methoxy-3-[2-(4-methyl-piperidin-1-yl)-ethoxy]phenyl}-2,3-dihydroisoindol-1-on,
6-Chlor-2-{4-methoxy-3-[2-(4-methyl-piperidin-1-yl)-ethoxy]phenyl} -2,3-dihydroisoindol-1-on,
5-Chlor-2-{4-methoxy-3-[2-(4-methyl-piperidin-1-yl)-ethoxy]phenyl}-2,3-dihydroisoindol-1-on,
7-Methyl-2- {4-methoxy-3-[2-(4-methyl-piperidin-1-yl)-ethoxy]phenyl}-2,3-dihydroisoindol-1-on,
6,7-Difluor-2-{4-methoxy-3-[2-(4-methyl-piperidin-1-yl)-ethoxy]phenyl}-2,3-dihydroisoindol-1-on,
5,6-Dichlor-2-{4-methoxy-3-[2-(4-methyl-piperidin-1-yl)-ethoxy]phenyl}-2,3-dihydroisoindol-1-on,
7-Fluor-2-{4-methoxy-3-[2-(piperidin-1-yl)-ethoxy]phenyl}-2,3-dihydroisoindol-1-on,
4-Fluor-2-{4-methoxy-3-[2-(piperidin-1-yl)-ethoxy]phenyl}-2,3-dihydroisoindol-1-on,
5,7-Dimethyl-2-{4-methoxy-3-[2-(4-methyl-piperidin-1-yl)-ethoxy]phenyl}-2,3-dihydroisoindol-1-on,
6,7-Dichlor-2-{4-methoxy-3-[2-(4-methyl-piperidin-1-yl)-ethoxy]phenyl}-2,3-dihydroisoindol-1-on,
5-Fluor-2- {4-methoxy-3-[2-(4-methyl-piperidin-1-yl)-ethoxy]phenyl} -7-trifluormethyl-2,3-dihydroisoindol-1-on,
7-Chlor-4,5-difluor-2- {4-methoxy-3-[2-(4-methyl-piperidin-1-yl)-ethoxy]phenyl}-2,3-dihydroisoindol-1-on,
4-Fluor-2- {4-methoxy-3-[2-(4-methyl-piperidin-1-yl)-ethoxy]phenyl} -7-trifluormethyl-2,3-dihydroisoindol-1-on,
5,7-Dichlor-2- {4-methoxy-3-[2-(4,4-dimethyl-piperidin-1-yl)-ethoxy]phenyl} -2,3-dihydroisoindol-1-on,
5,7-Dichlor-2-{4-methoxy-3-[2-(azepan-1-yl)-ethoxy]phenyl} -2,3-dihydroisoindol-1-on,
5,7-Dichlor-2-{4-methoxy-3-[2-(2-methyl-piperidin-1-yl)-ethoxy]phenyl}-2,3-dihydroisoindol-1-on,
6-{4-Methoxy-3-[2-(4-methyl-piperidin-1-yl)-ethoxy]-phenyl}-2-methyl-trifluormethyl-6,7-dihydro-pyrrolo[3,4-*b*]pyridin-5-on,
5,7-Dichlor-4-fluor-2-{4-methoxy-3-[2-(4-methyl-piperidin-1-yl)-ethoxy]phenyl}-2,3-dihydroisoindol-1-on
oder ein pharmazeutisch verträgliches Salz davon ist.

15. Verfahren zur Herstellung einer Verbindung wie in einem der Ansprüche 1 bis 14 beansprucht oder eines pharmazeutisch verträglichen Salzes davon, wobei das Verfahren umfasst:
(a) Umsetzen einer Verbindung der Formel (II): 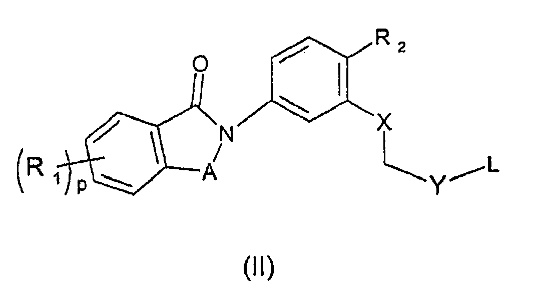 wobei R₁, R₂, p, A, X und Y wie für Formel (I) definiert sind und L eine Abgangsgruppe ist, mit einer Verbindung der Formel (III):
Z-H (III)
wobei Z wie für Formel (I) definiert ist; oder
(b) Umsetzen einer Verbindung der Formel (IV): 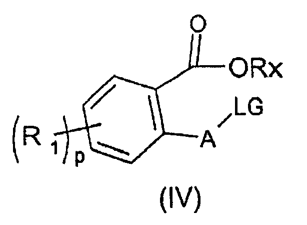 wobei Rx Alkyl ist und LG eine geeignete Abgangsgruppe ist, mit einer Verbindung der Formel (V) oder einem entsprechenden Salz: 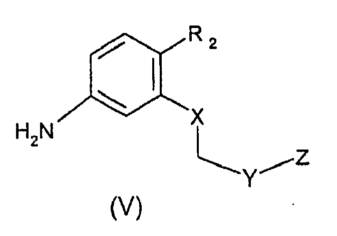 oder
(c) Umsetzen einer Verbindung der Formel (VI): 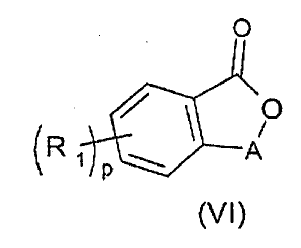
mit einer Verbindung der Formel (V) in Gegenwart von AlMe₃ oder einem ähnlichen oxophilen Reagenz, gefolgt von Behandeln des erhaltenen Amids unter dehydratisierenden Bedingungen, z.B. mit PPh₃ oder Dialkylazadicarboxylat;
und danach für jeweils Verfahren (a), Verfahren (b) oder Verfahren (c) gegebenenfalls gefolgt von:
- Entfernen jeglicher Schutzgruppen; und/oder
- Umwandeln einer Verbindung der Formel (I) in eine andere Verbindung der Formel (I); und/oder
- Bilden eines pharmazeutisch verträglichen Salzes.

16. Arzneimittel, umfassend eine Verbindung wie in einem der Ansprüche 1 bis 14 definiert oder ein pharmazeutisch verträgliches Salz davon und einen pharmazeutisch verträglichen Träger oder Exzipienten.

17. Verfahren zur Herstellung eines Arzneimittels nach Anspruch 16, wobei das Verfahren Mischen einer Verbindung wie in einem der Ansprüche 1 bis 14 definiert oder eines pharmazeutisch verträglichen Salzes davon und eines pharmazeutisch verträglichen Trägers oder Exzipienten umfasst.

18. Verbindung wie in einem der Ansprüche 1 bis 14 definiert oder ein pharmazeutisch verträgliches Salz davon zur Verwendung als eine therapeutische Substanz.

19. Verbindung wie in einem der Ansprüche 1 bis 14 definiert zur Verwendung bei der Behandlung einer ZNS-Störung wie Depression oder Angst.

20. Verwendung einer Verbindung wie in einem der Ansprüche 1 bis 14 beansprucht oder eines pharmazeutisch verträglichen Salzes davon zur Herstellung eines Medikaments zur Verwendung bei der Behandlung einer ZNS-Störung.

21. Verwendung gemäß Anspruch 22, wobei die ZNS-Störung Depression oder Angst ist.

## Revendications

1. Composé de formule (I) ou un de ses sels pharmaceutiquement acceptables : 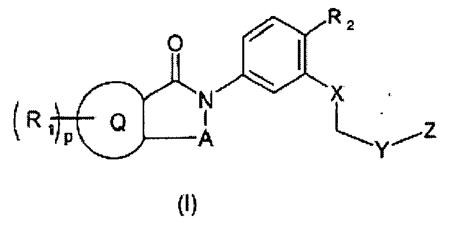 formule dans laquelle :
R₁ représente un groupe halogéno, cyano, alkyle en C₁ à C₆, cycloalkyle en C₃ à C₇, cycloalkyloxy en C₃ à C₇, alkoxy en C₁ à C₆, alkylthio en C ₁ à C ₆, hydroxy, amino, mono- ou di(alkyle en C₁ à C₆)amino, hétérocyclique tétra- à heptagonal lié par N, nitro, halogénalkyle en C ₁ à C ₆, halogénalkoxy en C₁ à C₆, aryle, -COOR₃, -COR₄ (dans lesquels R₃ et R ₄ représentent indépendamment un atome d'h ydrogène ou un groupe alkyle en C₁ à C ₆) ou -COR₅ (dans lequel R ₅ représente un groupe amino, mono - ou di(alkyle en C ₁ à C₆)amino ou hétérocyclique tétra- à heptagonal lié par N);
p est égal à 0, 1, 2 ou 3 ;
Q représente un groupe aromatique hexagonal ou un groupe hétéroaromatique hexagonal ;
A représente un groupe -(CH₂-CH₂)-, -(CH=CH)-, ou un groupe -(CHR₇)- dans lequel R ₇ représente un atome d'hydrogène, un groupe halogéno, hydroxy, cyano, nitro, alkyle en C₁ à C₆, cycloalkyle en C₃ à C₇, cycloalkyloxy en C₃ à C ₇, halogénalkyle en C₁ à C₆, alkoxy en C₁ à C₆, halogénalkoxy en C₁ à C₆, ou alkylthio en C₁ à C₆ ;
R₂ représente un atome d'hydrogène, un groupe halogéno, hydroxy, cyano, nitro, alkyle en C₁ à C ₆, alcanoyle en C₁ à C₆, cycloalkyle en C₃ à C₇, cycloalkyloxy en C₃ à C₇, halogénalkyle en C ₁ à C ₆, alkoxy en C ₁ à C₆, halogénalkoxy en C₁ à C₆, alkylthio en C₁ à C₆, amino, mono- ou di(alkyle en C₁ à C₆)amino ou hétérocyclique tétra- à heptagonal lié par N ;
X représente un atome d'oxygène ou de soufre, un groupe -CH₂- ou NR₈ dans lequel R₈ représente un atome d'hydrogène ou un groupe alkyle en C₁ à C₆ ;
Y représente une liaison simple, un groupe -CH₂-, -(CH₂)₂- ou -CH=CH- ; et
Z représente un groupe hétérocyclique lié par N, facultativement substitué, o u un groupe hétérocyclique tétra- à heptagonal, lié par C, contenant au moins un atome d'azote, ou bien Z représente un groupe -NR₉R₁₀ dans lequel R₉ et R₁₀ représentent indépendamment un atome d'hydrogène
ou un groupe alkyle en C₁ à C₆.

2. Composé suivant la revendication 1, dans lequel R₇ représente un atome d'hydrogène.

3. Composé suivant la revendication 1 ou la revendication 2, dans lequel A représente un groupe CH₂-.

4. Composé suivant l'une quelconque des revendications 1 à 3, dans lequel Q représente un groupe phényle.

5. Composé suivant l'une quelconque des revendications 1 à 4, dans lequel p est égal à 1, 2 ou 3 et le ou les groupes R₁ représentent des groupes halogéno (en particulier chloro ou fluoro), alkyle en C₁ à C₆ (en particulier méthyle) ou CF₃.

6. Composé suivant l'une quelconque des revendications 1 à 5, dans lequel, lorsque R₁ est fixé à la position marquée ci -dessous par un astérisque, R₁ représente un groupe fluoro :

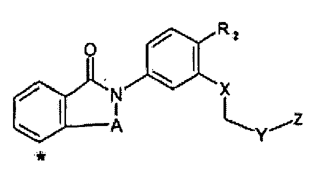

7. Composé suivant l'une quelconque des revendications 1 à 6, dans lequel, lorsque Q représente un groupe phényle et p est égal à 1, R₁ est fixé à la position marquée ci - dessous par un astérisque :

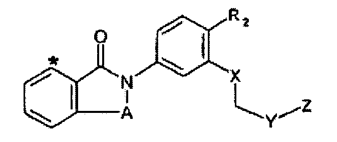

8. Composé suivant l'une quelconque des revendications 1 à 7, dans lequel, lorsque Q représente un groupe phényle et p est égal à 2 ou 3, R₁ est fixé à deux ou plus de deux des positions marquées ci-dessous par des flèches :

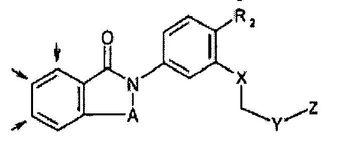

9. Composé suivant l'une quelconque des revendications 1 à 8, dans lequel R₂ représente un groupe alkoxy en C₁ à C₆, en particulier méthoxy.

10. Composé suivant l'une quelconque des revendications 1 à 9, dans lequel X représente un atome d'oxygène.

11. Composé suivant l'une quelconque des revendications 1 à 10, dans lequel Y représente un groupe -CH₂-.

12. Composé suivant l'une quelconque des revendication 1 à 11, dans lequel Z représente un hétérocycle tétra - à heptagonal lié par N, facultativement substitué, en particulier un groupe pipéridyle facultativement substitué.

13. Composé suivant la revendication 1, répondant à la formule (Ia) : 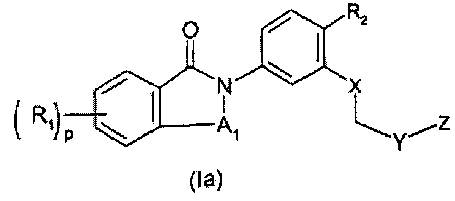 dans laquelle R₁, p, R₂, X, Y et Z répondent aux définitions de l'une quelconque des revendications 1 à 12 et A₁ représente un groupe -CH₂- ou -HC(Me)-.

14. Composé suivant la revendication 1, qui est
la 2-[4-méthoxy-3-(2-pipéridine-1-yl-éthoxy)phényl]-2,3-dihydroisoindole-1-one
la 6-fluoro-2-[4-méthoxy-3-(2-pipéridine-1-yl-éthoxy)-phényl]-2,3-dihydroisoindole-1-one
le chlorhydrate de 7 -bromo-2-{4-méthoxy-3-[2-(4-méthyl-pipéridine-1-yl)éthoxy]phényl}-2,3-dihydroisoindole-1-one
la 7 -chloro-2-{4-méthoxy-3-[2-(4-méthyl-pipéridine-1-yl)éthoxy]phényl}-3-méthyl-2,3-dihydroisoindole-1-one
la 2 -{4-méthoxy-3-[2-(4-méthyl-pipéridine-1-yl)éthoxy]-phényl}-7-triflorométhyl-2,3-dihydroisoindole-1-one
la 5, 7 -dichloro-2-{4-méthoxy-3-[2-(4-méthyl-pipéridine-1-yl)éthoxy]phényl}-2,3-dihydroisoindole-1-one
la 7-chloro-2-[4-méthoxy-3-(2-pipéridine-1-yl-éthoxy)-phényl]-2,3-dihydroisoindole-1-one
le chlorhydrate de 6chloro-2-[4-méthoxy-3-(2-pipéridine-1-yl-éthoxy)phényl]-2,3-dihydroisoindole-1-one
la 5-chloro-2-[4-méthoxy-3-(2-pipéridine-1-yl-éthoxy)-phényl]-2,3-dihydroisoindole-1-one
la 5,7 -dichloro-2-{4-méthoxy-3-[2-(cis-2,6-diméthylpipéridine-1-yl)éthoxy]phényl}-2,3-dihydroisoindole-1-one
la 7-chloro-2-{4-méthoxy-3- [2- (4-méthyl-pipéridine-1-yl)-éthoxy]phényl}-2,3-dihydroisoindole-1-one
la 6-chloro-2-{4-méthoxy-3- [2-(4-méthyl-pipéridine-1-yl)-éthoxy]phényl}-2,3-dihydroisoindole-1-one
la 5-chloro-2-{4-méthoxy-3- [2-(4-méthyl-pipéridine-1-yl)-éthoxy]phényl}-2,3-dihydroisoindole-1-one
la 7-méthyl-2-{4-méthoxy-3- [2-(4-méthyl-pipéridine-1-yl)-éthoxy]phényl}-2,3-dihydroisoindole-1-one
la 6,7 -difluoro-2-{4-méthoxy-3-[2-(4-méthyl-pipéridine-1-yl)éthoxy]phényl}-2,3-dihydroisoindole-1-one
la 5,6 -dichloro-2-{4-méthoxy-3-[2-(4-méthyl-pipéridine-1-yl)éthoxy]phényl}-2,3-dihydroisoindole-1-one
la 7 -fluoro-2-{4-méthoxy-3-[2-(pipéridine-1-yl)éthoxy]-phényl}-2,3-dihydroisoindole-1-one
la 4 -fluoro-2-{4-méthoxy-3-[2-(pipéridine-1-yl)éthoxy]-phényl}-2,3-dihydroisoindole-1-one
la 5, 7 -diméthyl-2-{4-méthoxy-3-[2-(4-méthyl-pipéridine-1-yl)éthoxy]phényl}-2,3-dihydroisoindole-1-one
la 6,7 -dichloro-2-{4-méthoxy-3-[2-(4-méthyl-pipéridine-1-yl)éthoxy]phényl}-2,3-dihydroisoindole-1-one
la 5-fluoro-2-{4-méthoxy-3-[2-(4-méthyl-pipéridine-1-yl)-éthoxy]phényl}-7-trifluorométhyl-2,3-dihydroisoindole-1-one
la 7 -chloro-4,5-difluoro-2-{4-méthoxy-3-[2-(4-méthyl-pipéridine-1-yl)éthoxy]phényl}-2,3-dihydroisoindole-1-one
la 4-fluoro-2-{4-méthoxy-3-[2-(4-méthyl-pipéridine-1-yl)-éthoxy]phényl}-7-trifluorométhyl-2,3-dihydroisoindole-1-one
la 5,7-dichloro-2-{4-méthoxy-3-[2-(4,4-diméthyl-pipéridine-1-yl)éthoxy]phényl}-2,3-dihydroisoindole-1-one
la 5,7 -dichloro-2-{4-méthoxy-3-[2-(azépan-1-yl)éthoxy]-phényl}-2,3-dihydroisoindole-1-one
la 5,7 -dichloro-2-{4-méthoxy-3- [2- (2-méthyl-pipéridine-1-yl)éthoxy]phényl}-2,3-dihydroisoindole-1-one
la 6 -{4-méthoxy-3-[2-(4-méthyl-pipéridine-1-yl)éthoxy]-phényl}-2-méthyl-4-triflorométhyl-6,7-dihydro-pyrrolo[3,4-b]pyridine-5-one
la 5,7 -dichloro-4-fluoro-2-{4-méthoxy-3-[2-(4-méthyl-pipéridine-1-yl)éthoxy]phényl}-2,3-dihydroisoindole-1-one
ou un de ses sels pharmaceutiquement acceptables.

15. Procédé pour la préparation d'un composé suivant l'une quelconque des revendications 1 à 14 ou d'un de ses sels pharmaceutiquement acceptables, procédé qui comprend :
(a) la réaction d'un composé de formule (II) : 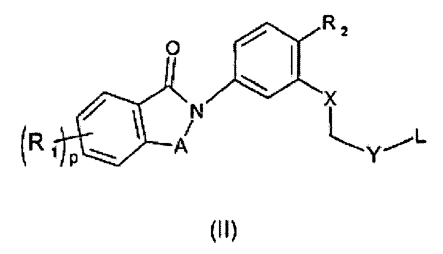 dans lequel R₁, R₂, p, A, X et Y sont tels que définis pour la formule (I) et L représente un groupe partant, avec un composé de formule (III) :
Z-H (III)
dans laquelle Z répond à la définition mentionnée pour la formule (I) ; ou
(b) la réaction d'un composé de formule (IV) : 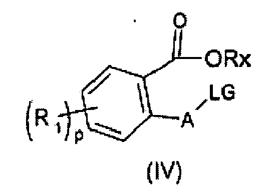 dans laquelle Rx représente un groupe alkyle et LG représente un groupe partant convenable, avec un composé de formule (V) ou un sel correspondant : 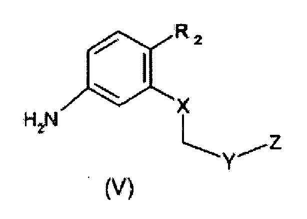 ou
(c) la réaction d'un composé de formule (VI) : 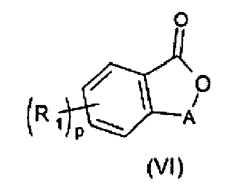
avec un composé de formule (V) en présence d'AlMe₃ ou d'un réactif oxophile similaire avec ensuite un traitement de l'amide résultant dans des conditions de déshydratation, par exemple avec PPh₃ et un azadicarboxylate de dialkyle ;
et ensuite, pour le procédé (a), le procédé (b) ou bien le procédé (c), facultativement :
• l'élimination de n'importe quels groupes protecteurs et/ou
• la conversion d'un composé de formule (I) en un autre composé de formule (I) ; et/ou
• la formation d'un sel pharmaceutiquement acceptable.

16. Composition pharmaceutique comprenant un composé tel que défini dans l'une quelconque des revendications 1 à 14 ou un de ses sels pharmaceutiquement acceptables, et un support ou excipient pharmaceutiquement acceptable.

17. Procédé pour la préparation d'une composition pharmaceutique telle que définie dans la revendication 16, procédé comprenant le mélange d'un composé tel que défini dans l'une quelconque des revendications 1 à 14 ou d'un de ses sels pharmaceutiquement acceptables, et d'un support ou excipient pharmaceutiquement acceptable.

18. Composé tel que défini dans l'une quelconque des revendications 1 à 14 ou un de ses sels pharmaceutiquement acceptables destiné à être utilisé comme substance thérapeutique.

19. Composé tel que défini dans l'une quelconque des revendications 1 à 14 destiné à être utilisé dans le traitement d'un trouble du SNC tel que la dépression ou l'anxiété.

20. Utilisation d'un composé suivant l'une quelconque des revendications 1 à 14 ou d'un de ses sels pharmaceutiquement acceptables dans la production d'un médicament destiné à être utilisé dans le traitement d'un trouble du SNC.

21. Utilisation suivant la revendication 22, dans laquelle le trouble du SNC est la dépression ou l'anxiété.
